# EUROPEAN PATENT SPECIFICATION

(11) **EP 0 279 688 B1**
(45) Date of publication and mention of the grant of the patent: **16.04.1997**
(21) Application number: 88301425.0
(22) Date of filing: 19.02.1988
(51) Int. Cl.: A61K 38/00, A61K 39/21, C12P 21/00, A61K 47/00

(54) **Methods and compositions for the use of HIV env polypeptides and antibodies thereto**
Verfahren und Zubereitungen für die Verwendung von HIV-env-Polypeptiden und Antikörpern
Méthodes et compositions pour l'utilisation de polypeptides env et anticorps anti-env de HIV

(30) Priority: 20.02.1987 US 16809; 01.06.1987 US 57061; 12.02.1988 US 155336
(43) Date of publication of application: 24.08.1988
(73) Proprietor: GENENTECH, INC., South San Francisco California 94080 (US)
(72) Inventor: Berman, Phillip W., San Mateo, CA 94402 (US); Gregory, Timothy J., Hillsborough, CA 94010 (US); Lasky, Lawrence A., Sausalito, CA 94965 (US); Nakamura, Gerald R., San Francisco, CA 94116 (US); Patzer, Eric J., Orinda, CA 94563 (US); Patton, John S., San Carlos, CA 94070 (US); Vitetta, Ellen S., Dallas, TX 75230 (US)
(74) Representative: Stuart, Ian Alexander

(56) References cited:
- EP-A- 0 187 041
- EP-A- 0 273 716
- WO-A-86/02383
- CELL, vol. 41, 1985; E.S. VITETTA et al., pp. 653-654/
- CANCER RESEARCH, vol. 46, July 1986; M. KRÖNKE et al., pp. 3295-3298/
- SCIENCE, vol. 231, 1986; J.S. McDOUGAL et al., pp. 382-385/
- BIOLOGICAL ABSTRACTS, vol. 80, 1985, Biological Abstracts Inc., Philadelphia, PA (US); A.H. FILIPOVICH et al., no. 51314/
- CHEMICAL ABSTRACTS, vol. 105, no. 21, 24 November 1986, Columbus, OH (US); D.A. WEIGENT et al., p. 563, no. 189109t/
- SCIENCE, vol. 233, 12 September 1986; D.M. BARNES, pp. 1149-1153/
- PROCEEDINGS OF THE NATL. ACADEMY OF SCIENCES USA, vol. 83, September 1986, Washington, DC (US); W.G. ROBEY, pp. 7023-7027/

## Description

This invention relates to methods for the use of Human Immunodeficiency Virus, or HIV, (HTLV-III) envelope (env) polypeptides, especially their use in suppressing undesirable immune responses and their inflammatory sequelae. The invention also is concerned with the use of HIV env variants and antibodies to HIV env in the vaccination and treatment of HIV-infected patients, in the diagnostic assay of test samples and for other therapeutic indications. In particular, this invention is concerned with the treatment of autoimmune diseases and the suppression of transplantation immunity. The invention also relates to immunotoxins and other immunoconjugates and their use in killing cells infected with HIV or other viruses which encode a cell surface antigen.

Inflammatory responses associated with various immune disorders are typically divided into four classes. However, numerous autoimmune diseases whose treatment is included within the scope herein are of uncertain origin and therefore resistant to classification, e.g. myasthenia gravis, Graves disease, autoimmune sequelae to Chagas disease, and juvenile-onset diabetes. Class I responses are reaginic or allergic reactions characterizing atopy or anaphylaxis. Class II responses are dependent upon cytotoxic antibody and are associated, for example, with autoimmune hemolytic anemia and thrombocytopenia attendant to systemic lupus erythematosus (SLE). Class III responses are characterized by chronic generation of immune complexes containing IgG and/or IgM. Class IV responses, associated with delayed hypersensitivity, are mediated by cytokines and T-lymphocytes and are typically found in tuberculosis, sarcoidosis, polymyositis, granulomatosis and vasculitis.

Rheumatoid arthritis (RA) is a serious class III disorder. Synovitis is the characteristic tissue reaction in RA, wherein the normally thin connective tissue is replaced by hyperproliferating synovial fibroblasts, and the synovium is invaded by an infiltrate of lymphocytes, macrophages and other immune cells. The turnover of complement components is increased in rheumatic synovial fluid, and complement cleavage products, especially C5a, are present in rheumatoid synovial fluid. This is significant because C5a and other derivatives of complement contribute to the activation of immune cells and the production of inflammatory cytokines. The fluid also contains hydrolytic enzymes (including collagenase) derived from inflammatory cells, together with kinins and LTB4.

Various mechanisms have been proposed to account for the degree of tissue destruction characteristic of rheumatoid arthritis. These mechanisms involve a complex interplay of activation and suppression mechanisms which modulate the type and magnitude of the inflammatory response to RA. These mechanisms involve arachidonic acid metabolites, especially prostaglandin E2 produced by leukocytes and rheumatoid synovia, biologically active amines such as histamine and serotonin, complement cleavage products, eosinophil chemotactic factors, kinins, interleukins and proteolytic enzymes, especially collagenase or procollagenase activating enzymes.

One commonly accepted postulate for induction of RA holds that an undefined antigen is chronically deposited in the synovium where it is phagocytized by the Type A synovial cells. Presentation of the antigen to B cells induces their differentiation to plasma cells, which then produce antibodies, rheumatoid factors and cytokines. Antigen activation of T lymphocytes triggers lymphokine synthesis, blastogenesis and the subsequent generation of antigen-specific helper and cytotoxic T-cells. The combination of antibody with antigen, as well as combination of antigen-antibody complexes with rheumatoid factors, or self-association of rheumatoid factors, activates complement as well as the kinin-forming system by means of activating Hageman factor. This results in the production of proinflammatory products such as C5a, arachidonic acid metabolites, kinins, and fibrinopeptides, which diffuse into the synovial fluid and to synovial blood vessels. These agents enhance vascular permeability and attract polymorphonuclear leukocytes and macrophages. Polymorphonuclear leukocytes ingest the abundant immune complexes in the fluid, release lysosomal and other destructive enzymes, and generate superoxide anions. This causes destruction of hyaluronate polymers in the joint fluid, as well as injury to cartilage. Cytokine production in the synovium leads to the further accumulation of macrophages, other immune cells and rheumatoid synovial fibroblasts. These cell types can all contribute further to the cycle of immune cell recruitment, activation and production of immune mediators (e.g., cytokines) which is characteristic of RA and leads to the perpetuation of the inflammatory state and the resulting tissue destruction.

The unique structure of the joint space is important, as enzymes present in synovial fluid or released and synthesized locally by the cells constituting the proliferative synovial lesion contribute to the pathology evident in articular structures. The cartilege-degrading lysosomal enzymes collagenase and elastase are primarily derived from inflammatory cells (immune cells and rheumatoid synovial fibroblasts). Proteinase released by dying cells may aid in superficial cartilage destruction by virtue of their role in uncrosslinking collagen fibrils, thus increasing their susceptibility to enzymatic degradation. Macrophages in the synovium produce prostaglandins, hydrolytic enzymes, collagenase, plasminogen activator, and IL-1. Collagenase synthesis by macrophages or rheumatoid synovial fibroblasts is greatly enhanced in the presence of IL-1 and other inflammatory proteins. In addition to collagenases, lysosomal proteinases can degrade aggregates of proteoglycans, and, once released from cartilage, these solubilized components are then sensitive to further enzymatic attack.

Persistent antigen activation or disordered regulation of T and B cell activation leads to a chronic inflammatory response in the synovium. Continued cellular proliferation and influx lead to synovial proliferation and its invasion into surrounding structures. Diffusion of collagenase, PGEs, hydrolytic enzymes, and cytokines into cartilage and bone results in erosion of these tissues. Rheumatoid arthritis thus illustrates the devastating local tissue destruction that results from chronic inflammatory reactions produced by delayed hypersensitivity types of immune responses.

Current therapy for RA includes bed rest, application of heat, and drugs. Salicylate is the currently preferred drug, particularly as other alternatives such as currently available immunosuppressive agents and adrenocorticosteroids can cause greater morbidity than the underlying disease itself. Nonsteroidal anti-inflammatory drugs are available, and many of them have effective analgesic, antipyretic and anti-inflammatory activity in RA patients. These include indomethacin, phenylbutazone, phenylacetic acid derivatives such as ibuprofen and fenoprofen, naphthalene acetic acids (naproxen), pyrrolealkanoic acid (tometin), indoleacetic acids (sulindac), halgenated anthranilic acid (meclofenamate sodium), piroxicam, zomepirac and diflunisal. These are not generally considered any more effective than aspirin, but may be better tolerated by some patient classes.

Other drugs for use in RA include antimalarials such as chloroquine, gold salts and penicillamine. These alternatives frequenty produce severe side effects, including retinal lesions and kidney and bone marrow toxicity. Methotrexate, an immunosuppressive agent, has been used only in the treatment of severe and unremitting RA because of its toxicity. Corticosteroids also are responsible for undesirable side effects and are not well tolerated in many RA patients.

Methods and compositions are needed for the effective therapy of chronic inflammatory diseases or disorders associated with class I-IV response, in particular for the suppression of responses associated with ulcerative colitis (inflammatory bowel disease), RA or SLE (systemic lupus erythematosus) myasthenia gravis, Graves disease, Chagas disease and juvenile-onset diabetes.

This invention also is concerned with suppressing the immune rejection of, or by, transplanted cells, organs or tissues. Transplant rejection has been suppressed by the use of immunosuppressive drugs such as cyclosporin antibiotics and steroids. Such drugs exert extensive and undesirable side effects. In another approach, monoclonal antibodies have been used to target and neutralize lymphocyte subsets believed to be involved in transplant rejection. These antibodies are murine in origin. As is the case with murine monoclonal antibodies in general, these antibodies exhibit low affinity for their target T cell subsets. They also pose a risk of inducing murine hypersensitivity in treated patients and, because murine constant regions are capable of activating human complement, the anti-T subset antibodies pose the risk of T cell lysis and depletion. Compositions and methods are needed for the treatment of graft rejections which do not employ murine immunoglobulins.

Acquired immunodeficiency syndrome (AIDS) is caused by a retrovirus identified as human immuno-deficiency virus (HIV)(1-6). A number of immunologic abnormalities have been described in AIDS including abnormalities in B-cell function, abnormal antibody response, defective monocyte cell function,impaired cytokine production (7-10), depressed natural killer and cytotoxic cell function (11), and defective ability of lymphocytes to recognize and respond to soluble antigens (12). Other immunologic abnormalities associated with AIDS have been reported (13,14). Among the more important immunologic defects in patients with AIDS is the depletion of the T4 helper/inducer lymphocyte population (1,2,9,10).

In spite of the profound immunodeficiency observed in AIDS, the mechanism(s) responsible for immunodeficiency are not clearly understood. Several postulates exist. One accepted view is that defects in immune responsiveness are due to selective infection of helper T cells by HIV resulting in impairment of helper T cell function and eventual depletion of cells necessary for a normal immune response (1-6,15). Recently in vitro and in vivo studies showed that HIV can also infect monocytes which are known to play an essential role as accessory cells in the immune response (16,17). HIV may also result in immunodeficiency by interfering with normal cytokine production in an infected cell resulting in secondary immunodeficiency as for example, IL-1 and IL-2 deficiency (18). An additional means of HIV-induced immunodeficiency consists of the production of factors which are capable of suppressing the immune response (19). None of these models resolves the question of whether a component of HIV per se, rather than infection by replicative virus, is responsible for the immunologic abnormalities associated with AIDS.

The HIV env protein has been extensively described, and the amino acid and RNA sequences encoding HIV env from a number of HIV strains are known (34). The HIV virion is covered by a membrane or envelope derived from the outer membrane of host cells. The membrane contains a population of envelope glycoproteins (gp160) anchored in the membrane bilayer at their carboxyl terminal region. Each glycoprotein contains two segments. The N-terminal segment, called gp120 by virtue of its relative molecular weight of about 120 kD, protrudes into the aqueous environment surrounding the virion. The C-terminal segment, called gp41, spans the membrane. gp120 and gp41 are covalently linked by a peptide bond that is particularly susceptible to proteolytic cleavage. For the purpose herein, HIV env includes all forms of gp120, e.g. fusions such as gp160 or gp120 fused to N-terminal fragments of gp41, variantly glycosylated or unglycosylated HIV env and T cell-binding fragments of HIV env. HIV env and its variants are conventionally prepared in recombinant cell culture. For example, see EP Publication No. 187041. Henceforth, gp120 prepared in recombinant cell culture is referred to as rgp120. Recombinant synthesis is preferred for reasons of safety and economy, but it is known to purify HIV env from viral cultures and such env preparations are included within the definition of HIV env.

Current AIDS therapy which is directed towards protecting uninfected cells, consists of oral dosing about every four hours with nucleoside analogues (such as AZT and DDC) which inhibit viral RNA replication. Although these drugs inhibit viral replication at concentrations of 50-500 µm, at higher concentrations (⁻1 mM) they also inhibit the DNA polymerase of healthy cells which is required for cell division (Mitsuya and Broder, 1985). The current therapy requires very large doses of drugs (up to a gram/day). Because the drugs are taken orally and in a form that is absorbed by all cells, the entire body is exposed to them. Toxicity is a serious limitation to their use; anemia being one of the most severe side effects. Because nucleoside derivatives must be phosphorylated before they can be incorporated into DNA (and express their chain disrupting activity) they require kinases which are not present in equal amounts in all cells susceptible to viral infection. Thus the oral nucleoside analogue therapy, which is ineffective against already infected cells, is only able to protect those susceptible cells which can convert high concentrations of nucleosides into nucleotides (i.e., dividing cells). For these reasons this therapy is limited and the progression of the disease is only slowed.

At least two kinds of immune system cells are infected by HIV (human immunodeficiency virus), monocytes, and T-lymphocytes (Streicher, 1986). Only those monocytes and T-cells which have the CD4 receptor are thought to be infected by HIV (McDougal et al., 1986). A conserved region of the HIV viral coat protein (gp160) binds to the CD4 receptor which undergoes internalization and carries the RNA virus into the cell. Once inside the cell the virus makes a DNA copy of its RNA with its enzyme reverse transcriptase. Nucleoside analogues protect cells by serving as chain breakers in the transcription of viral RNA. They are incorporated into the growing polymer but lack the functional group necessary to bind the next nucleoside into the chain, thus the chain is interrupted and therefore non-functional.

Before a nucleoside analogue can be incorporated into a strand of nucleic acid it must be phosphorylated by a kinase (converted to a nucleotide) (Furman et al., 1986). T-cells which are capable of rapid division possess high levels of kinases to phosphorylate antiviral drugs like AZT and DDC. Monocytes which divide only under special circumstances, possess relatively low levels of the requisite kinases and the nucleoside derivatives do not protect them against infection. The simple solution would be to administer the phosphorylated form of the nucleotide analogues. These, however, are not transported across cell membranes. Thus the current nucleoside analogue therapy is ineffective in protecting monocytes from HIV infection.

Lyerly et al., PNAS 84, 4601 (1987) have shown that uninfected CD4⁺ lymphocytes that have bound gp120 become targets for antibody-dependent cellular cytotoxicity by a goat serum raised against native gp120. However, human sera that binds to gp120-adsorbed cells failed to direct their destruction in the presence of complement. In contrast, these sera were potent mediators of antibody dependent cellular cytotoxicity.

Monoclonal and polyclonal antibodies have been developed which are specific for HIV-encoded proteins - see for example WO-A-8604613.

Envelope glycoproteins of LAV and HIV inducing neutralizing antibodies are disclosed in WO-A-8602383 and by Robey et al., PNAS 83, 7023 (1986).

Immunotoxins (ITs) composed of cell-reactive antibodies coupled to the A chain of the ricin toxin (IT-As), have been used to specifically kill a variety of target tumor cells. Kronke et al., in Cancer Res, 46(7) 3295 (1986) disclose killing of HTLV-I infected leukemic T cells by monoclonal anti-interleukin 2 receptor antibody-ricin A chain conjugates. See also Kronke in Blood 65(6), 1416 (1985).

Shouval et al., U.S. Patent No. 4,714,613, discloses a method of suppressing the growth of hepatitis B virus infected hepatocytes or hepatoma cells which express hepatitis B surface antigen on the surface, wherein the surface antigen is coded for by the DNA of the HBV. The Shouval et al. method comprises administering to the infected cells, complement fixing monoclonal antibody against hepatitis B surface antigen.

Desiderata which may be achievable by the present invention include:
i) to provide an improved method of treating immunoinflammatory disorders.
ii) to provide an improved method of treating AIDS with reduced side effects.
iii) to provide a method of killing non-tumorigenic cells which express virally encoded antigens on their cell surface.

HIV env is useful in the therapy of immunoinflammatory responses and disorders. The desiderata of the invention may be accomplished by administering an HIV env preparation comprising the TCB domain to a patient having an immunoinflammatory response in an amount sufficient to suppress the immunoinflammatory response.

In an embodiment of this invention the HIV env polypeptide employed is the portion of HIV env that is responsible for binding of the polypeptide to the T4 cell surface marker of T helper cells. This portion of the HIV env sequence, which is referred to as the TCB domain, has been located in an unexpected region of the molecule including residues 400 to 465. The TCB domain, including variant analogues thereof, in HIV env also is useful for the treatment of immunoinflammation. The TCB domain is useful as well in diagnostic assays for TCB domain-neutralizing antibody in patient samples.

A monoclonal antibody is provided that is capable of blocking or binding the TCB domain. This antibody appears to be directed to an epitope that is located adjacent to the sequence in the TCB domain that binds to the T4 cell surface marker. This is demonstrated by the ability of the antibody to at least partially block the binding of recombinant gp120 to the T4 cell surface marker, probably by sterically hindering access of the TCB domain to its binding site on the T4 surface antigen. This antibody also is useful in passively immunizing patients infected with HIV with the additional advantage of binding any free HIV env that is exerting an immunosuppressive effect on such patients.

Immunotoxins comprising a monoclonal antibody specifically binding a region of the TCB domain of an HIV env and a toxin such as ricin A chain linked to said monoclonal antibody are provided to target virally encoded proteins. Then, to the extent that the proteins which have bound the toxic conjugate are taken up by infected cells such as helper T cells, the cells are killed. The invention thus makes available a method of killing virally infected cells comprising administering to a patient a therapeutically effective amount of an immunotoxin comprising a monoclonal antibody linked to a toxin which specifically binds virally encoded protein (TCB domain) on the cell surface thereby killing the infected cells.

The TCB domain of HIV env which is free of HIV immune epitopes ordinarily flanking the C-terminal or N-terminal ends of the HIV env is conjugated to haptens or polypeptides capable of binding a cell population against which a cytotoxic T cell cytolytic response is desired. These conjugates are capable of binding to the T4 receptor of helper lymphocytes. They may have particular utility as antitumor agents or in the treatment of allergic responses.

HIV env is defined as envelope protein of Human Immunodeficiency Virus as described above, together with its immunosuppressive amino acid sequence variants and derivatives produced by covalent modification of HIV env or its variants in vitro. Variants include HIV env in which one or more residues have been substituted in the env amino acid sequence, deletions of one or more residues in the env sequence and insertions of one or more residues therein.

The principal deletion variants of interest are those in which immune epitopes other than the TCB domain have been deleted, Such deletion variants, as well as further substitutional variants are described in more detail below. Another class of variants are those in which a polypeptide heterologous to HIV env is fused to the amino or carboxyl terminus of the TCB domain. Alternatively, HIV env or its TCB fragment is covalently bonded to the heterologous polypeptide or to a hapten. Another class of TCB domain variants is produced by fusing the TCB domain to HIV immune epitopes other than those which may normally flank the TCB domain in native HIV env, in particular other than the TCB domain C-terminal flanking sequence FRPGGGDMRDNWRSELYKYKV.

The fusions of the HIV env TCB domain with an immunogenic hapten or polypeptide are useful as vaccine components for the immunization of patients against HIV infection. Fusions of the hapten or heterologous polypeptide with HIV env or its active fragments are useful in directing cytotoxic T cells against target cells where the hapten or heterologous polypeptide is capable of binding to a target cell surface receptor or other binding partner. For example, membrane-bound transforming growth factor-α (TGF-α) is present on the surface of many solid (non-hematopoietic) neoplastic tumors. Antibodies capable of binding TGF-α are known. Such antibodies are linked to HIV env or its TCB domain-containing fragments, e.g. by covalent crosslinking (33), or by expression in recombinant cell culture as an N- or C-terminal fusion with HIV env or its TCB domain-containing fragment. The latter alternative is accomplished by obtaining DNA encoding the TGF-α antibody in the fashion generally described in EP 125,023A. This DNA is ligated by the use of appropriate adaptors to the DNA encoding the amino or carboxyl terminus of gp120 or its TCB domain-containing fragments, inserted into a mammalian cell expression vector such as is described in EP 187,041A and the vector used to transform a suitable host cell such as CHO cells or others described in EP 187,041A. Optionally, DNA encoding the variable region of the TGF-α antibody is ligated to DNA encoding the TCB domain in place of the human constant regions described in EP 184,187; EP 194,276A; EP 171,496A; or Bouliamne et al.(32). The resulting fusions are recovered, e.g. by adsorption to immobilized TGF-α or immobilized antibody directed against the constant region of the TGF-α antibody when the constant region is retained, formulated into a therapeutically acceptable vehicle, e.g. physiological saline, and administered to patients bearing TGF-α expression neoplasms. The fusions will direct cytotoxic T cells to tumors having TGF-α cell surface antigens, resulting in lysis of the tumor cells. Similarly, the TCB domain is fused to an antigen against which a patient is raising an undesirable immune response, e.g. as in an allergic or transplantation rejection episode, and administered to a patient in doses sufficient to target an autoimmune cytotoxic T cell response to the clonal cells responsible for the undesirable response.

The TCB domain of HIV env is believed to include the region extending from about nucleotide 7006 to about nucleotide 7203 of the HIV strain described in EP 187,041A, or the equivalent region of other HIV strains (the same nucleotide and amino acid residue numbers may not be applicable in other strains where upstream deletions or insertions change the length of the viral genome and HIV env, but the region encoding this portion of the TCB domain in each strain is readily identified by amino acid sequence homology. The TCB domain includes the sequence: 
TEGSNNTEGSDTITLPCRIKQFINMWQEVGKAMYAPPISGQIKCSSNITGLLLTRDGGNNNNESEI
|         |         |         |         |         |         |    |
400       410       420       430       440       450       460  465
 particularly the sequence falling within residues 411-454, 416-443 and 420-442. In Table 1 below, the amino acid sequences of the TCB domains from a plurality of HIV stains are shown in the right column under "origin" while predetermined variant TCB domains are shown in the left column. The variant identified as "Δ3" (variant 1) led to the identification of this portion of the TCB domain for HIV. Deletion of the Δ3 residues from rgp120 by site-directed M13 mutagenesis and expression of the variant in recombinant CHO cell culture otherwise as shown in EP 187,041 resulted in the abolition of the T4 binding capacity of rgp120, notwithstanding that the rgp120 was indistinguishable from rgp120 based on assays such as binding by selected AIDS patient sera. Surprisingly, the Δ3 region is not highly conserved among a number of HIV strains, as can be appreciated by comparison with the homologous sequences from other HIV strains than BH10 shown in Table 1, and the TCB domain as a whole contains regions of HIV env characterized as highly variable (34). It would have been expected that only highly conserved regions would have the constancy suitable to ensure that the virus could always recognize the T4 receptor and thereby infect permissive host cells, but this hypothesis has not withstood investigation. For example, deletion of the first 29 amino terminal residues of HIV env had no effect on T4 binding of rgp120, despite the fact that resides 5-31 are highly conserved among various HIV strains.

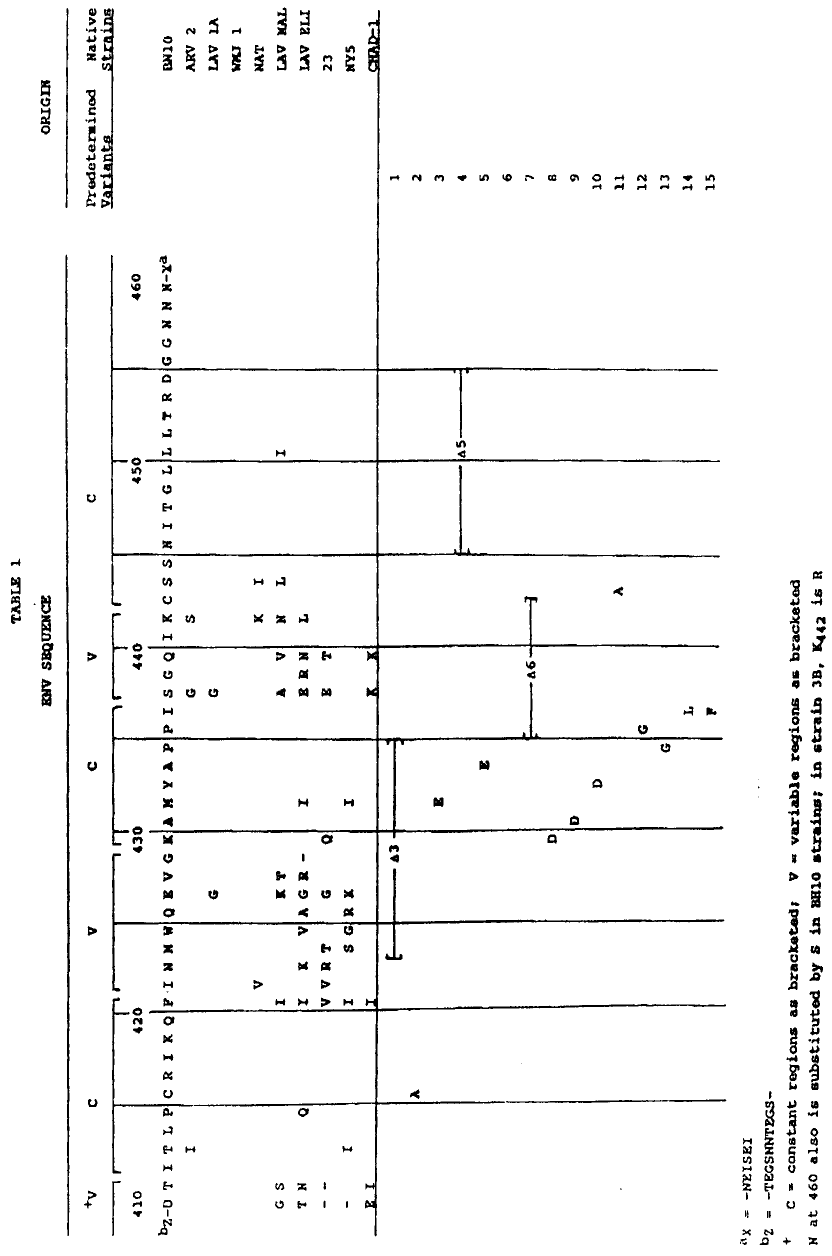

The variants designated in Table 1 by numbers 2-15 are expected to modify or eliminate the T4 binding characteristics of rgp120. For example, substitution for alanine by aspartic acid at position 431 reduced the binding of the variant to about 10-15% of that exhibited by rpg120 bearing the native sequence. However, substitution of lys₄₃₀ with glutaminyl, tyr₄₃₃ with phenylalanyl, and pro₄₃₅pro₄₃₆ with valylvalyl did not result in any change in rgp120 binding detectable by our assays. Accordingly, each variant will be screened by routine assays in order to determine its activity. In the light of the role of the Ala₄₃₁ residue, substitutions, deletions or insertions at this site are particularly preferred, including substitutions by histidine, tryptophan, proline, phenylalanine, glycine, leucine, isoleucine, methionine, valine, serine, threonine, tryosine, glutamine, asparagine, lysine, glutamic acid, cysteine and arginine, or insertions by such residues or aspartic acid adjacent to Ala₄₃₁. Such variants, as well as the Δ3 variant, are useful in preparing HIV env antigens for use in diagnostic assays or vaccines. HIV env which is free of the TCB domain is particularly useful in vaccines because it is not immunosuppressive but nonetheless retains as many of the remaining gp120 eptitopes as are desired, thereby leading to higher titers of anti-HIV and prolongation of the titers. Other variants will be apparent to the ordinary artisan. For example, substitutions are made within residues 416-442 in order to identify variants that act as HIV env agonists. Deletions or insertions also can be introduced into the TCB domain and evaluated for agonist activity. "Agonist" in this context means that the HIV env activity of the variant is enhanced in comparison to the native molecule (for example the variant exerts higher affinity for T4 than does native HIV env). It will be understood, however, that this activity is antagonistic to infectivity by HIV. HIV env agonist analogues are useful as agents for the treatment of undesirable immunoinflammatory events in the same fashion as described herein for intact or native HIV env. Agonist analogues are administered to AIDS or ARC patients in the treatment of HIV infection, optionally together with tumor necrosis factor α, tumor necrosis factor β, an interferon, and/or other therapeutic agents for the treatment of HIV infections, e.g., AZT.

HIV env agonist activity is measured in the fluorescence activated cell sorter system described in the example below by measuring the ability of the agonist to compete with labelled rgp120 or native gp120, or with fluorescein isothiocyanate-conjugated anti-T4A antibody, for binding to the T4 receptor. This ability of the candidate variant to compete with the labelled conjugates for T cell binding in PBMC is one measure of its activity: The greater the competition with labelled conjugates by the varianet for the T4 binding site, the greater is the variant's agonist activity.

The HIV env TCB domain or its variants are produced by methods known per se. One method is to synthesize the TCB domains by in vitro procedures such as Merrifield synthesis. Another method is to prepare the domains in recombinant cell culture. It is practical to prepare DNA encoding the domain by conventional in vitro methods, providing suitable 5ʹ and 3ʹ adaptors or linkers to facilitate ligation into conventional vectors for bacterial expression. In one preferred embodiment the DNA encoding the domain is ligated at its 5ʹ end to DNA encoding a signal sequence recognized by the host cell. Examples of such signals are well known and include the STII, alkaline phosphatase, lpp, and penicillinase signals. Certain heterologous signals are also recognized by bacteria, e.g. mammalian or yeast signals. Since the DNA encoding such signals is only about 45 to 75 nucleotides in length, one would prepare and ligate signal DNA to the 5ʹ end of the DNA encoding the TCB domain, ligate the fusion gene into a conventional vector, e.g. pBR322, transfect host E. coli, culture the cells and determine the amount of TCB domain secreted into the periplasm of the host cell. Alternatively, the TCB domain is synthesized in recombinant cell culture as a secreted or non-secreted fusion which is recovered.

An antibody capable of blocking or binding the TCB domain is obtained by immunizing mice such as Balb/c or, preferably C57 black/6, against gp120 and screening for a clonal antibody that, when preincubated with gp120, prevents its binding to the T4 T cell marker. The 5C2E5 antibody is an example of such an antibody, but is not unique since other antibodies having the same qualitative activity can be obtained by the method described herein. "Qualitative" activity means that the antibody binds to the T4 binding site of HIV env or a flanking region, in the latter situation binding in such a way as to sterically hinder the binding of rgp120 or HIV env to the T4 site. A tryptic digest of the 44 residue TCB domain showed that the 5C2E5 antibody bound the peptide spanning residues 420-430. This domain is thus included in the TCB domain or is immediately adjacent thereto. The 5C2E5 antibody was found in about 1 out of 500 myeloma fusions with murine B cells recovered from mice immunized against gp120. An additional blocking antibody is 7F11, which binds to the peptide spanning residues 430-459.

The TCB domain binding characteristics of antibody 5C2E5 are defined as the ability to inhibit the binding of rgp120 to the T4 receptor of helper lymphocytes. Monoclonal antibodies other than 5C2E5 which are capable of doing so are included within the scope of this definition, despite any differences in affinity, immunoglobulin class, species of origin, or exact TCB sequence, as are antibodies which are expressed in recombinant cell culture or that are predetermined amino acid sequence variants of the 5C2E5 antibody, e.g. chimeras of the variable region of the 5C2E5 antibody and the human constant region.

The antibody described herein is recovered from hybridoma cell cultures by conventional methods for purification of IgG or IgM as the case may be that heretofore have been used to purify these immunoglobulins from pooled plasma, e.g. ethanol or polyethylene glycol precipitation procedures. The purified antibody is sterile filtered and optionally conjugated to a cytotoxin such as ricin for use in AIDS therapy or conjugated to a detectable marker such as an enzyme or spin label for use in diagnostic essays of HIV in test samples.

A new application of immunotoxins has been discovered. Surprisingly, it has been found that an immunotoxin specifically binding virally induced surface antigen of non-tumorigenic virus infected cells, kills the infected cells. Specifically immunotoxins binding a region of the TCB domain, kill HIV infected cells.

Described below is a treatment designed to kill cells that are already infected and are actively producing new virus. Killing is accomplished by an immunotoxin that binds to viral coat protein which is expressed on infected cells. The immunotoxin is then internalized and kills the cell. Infected cells that have incorporated viral genome into their DNA but are not synthesizing viral protein (i.e., cells in which the virus is latent) may not be susceptible to killing by immunotoxin until they begin to synthesize virus.

It has been discovered that cells infected with HIV that are actively producing virus, express viral coat protein (gp160) on their plasma membrane. This molecule represents an infected-cell-specific antigen that can be used to target and kill infected cells. Monoclonal antibodies specific for a conserved region (strain independent) on the gp160 can be used as targeting molecules to deliver toxins to infected cells. Additionally or alternatively, a "cocktail" of immunotoxins against nonconserved regions is used. In addition the toxin-antibody conjugate can bind to circulating viruses or viral coat protein which will then effect killing of cells that internalize virus or coat protein.

The subject invention provides a highly selective method of destroying HIV infected cells. Because the antibodies specifically bind antigen not present in the cells when not infected, the method permits the antibody to pinpoint with great accuracy cells to be destroyed. Unlike tumor treatment with immunoconjugates where loss of antigen occurs and resistance develops, with active virus infection antigen expression always occurs.

While not wishing to be constrained to any particular theory of operation of the invention, it is believed that the expression of the target antigen on the infected cell surface is transient. The antibodies must be capable of reaching the site on the cell surface where the antigen resides and interacting with it. After the antibody complexes with the antigen, endocytosis takes place carrying the toxin into the cell.

The immunotoxins are particularly helpful in killing monocytes/macrophages infected with the HIV virus. In contrast to the transient production of virus from T cells, macrophages produce high levels of virus for long periods of time. Current therapy is ineffective in inhibiting the production of new viruses in these cells.

Not all monoclonal antibodies specific for gp160 make highly cytotoxic immunotoxins when coupled to ricin A chain (IT-As). An assay was developed which is used to predict the ability of an antibody to function as part of an IT-A. Using this assay, a variety of anti-HIV antibodies were screened for their ability to make effective IT-As against HIV-infected cells. Results demonstrate that only some HIV-specific antibodies make effective IT-As. These antibodies are purified, coupled to ricin A chain and used to specifically kill HIV-infected cells. Advantageously the antibodies cross react with several (or all) strains of HIV.

Immunotoxins can be made in a variety of ways (see detailed discussion below). Several crosslinking reagents can be used to make the conjugate to yield stable conjugates.

In a preferred embodiment, ricin A chain is deglycosylated or produced without oligosaccharides, to decrease its clearance by irrelevant clearance mechanisms (i.e., the liver). In another embodiment, whole ricin (A chain plus B chain) is conjugated to antibody if the galactose binding property of B-chain can be blocked ("blocked ricin").

In a further embodiment toxin-conjugates are made with Fab or (F(Abʹ)₂ fragments. Because of their relatively small size these fragments can better penetrate tissue to reach infected cells.

### Antibodies

In accordance with this invention, monoclonal antibodies specifically binding an epitope of gp160 or antigenically active, cell surface-exposed fragments thereof (for example epitopes chosen frm gp120, gp41, the TCB and the like) were isolated from continuous hybrid cell lines formed by the fusion of antigen-primed immune lymphocytes with myeloma cells. Advantageously, the monoclonal antibodies of the subject invention which bind gp160, bind the domain of this protein which is exposed on the cell surface. In another embodiment of the invention, polyclonal antibodies specifically binding gp160 are used.

The antibodies of the subject invention are obtained through screening. An assay is used for screening monoclonal antibodies for their cytotoxic potential as ricin A chain containing immunotoxins. The assay involves treating cells with dilutions of the test antibody followed by a Fab fragment of a secondary antibody coupled to ricin A chain ("indirect assay"). The cytotoxicity of the indirect assay is compared to that of the direct assay where the monoclonal antibody is coupled to ricin A chain. The indirect assay accurately predicts the potency of a given monoclonal antibody as an immunotoxin and is thus useful in screening monoclonal antibodies for use as immunotoxins - see also Vitetta et al., Science 238: 1098-1104 (1987), and Weltman et al., Cancer Res, 47, 5552 (1987), hereby incorporated by reference.

Monoclonal antibodies are highly specific, being directed against a single antigenic site. Furthermore, in contrast to conventional antibody (polyclonal) preparations which typically include different antibodies directed against different determinants (epitopes), each monoclonal antibody is directed against a single determinant on the antigen. Monoclonal antibodies are useful to improve the selectivity and specificity of diagnostic and analytical assay methods using antigen-antibody binding. A second advantage of monoclonal antibodies is that they are synthesized by the hybridoma culture, uncontaminated by other immunoglobulins. Monoclonal antibodies may be prepared from supernatants of cultured hybridoma cells or from ascites induced by intra-peritoneal inoculation of hybridoma cells into mice.

The hybridoma technique described originally by Kohler and Milstein, Eur. J. Immunol. 6, 511 (1976) has been widely applied to produce hybrid cell lines that secrete high levels of monoclonal antibodies against many specific antigens.

The route and schedule of immunization of the host animal or cultured antibody-producing cells therefrom are generally in keeping with established and conventional techniques for antibody stimulation and production. Applicants have employed mice as the test model although it is contemplated that any mammalian subject including human subjects or antibody producing cells therefrom can be manipulated according to the processes of this invention to serve as the basis for production of mammalian, including human, hybrid cell lines.

After immunization, immune lymphoid cells are fused with myeloma cells to generate a hybrid cell line which can be cultivated and subcultivated indefinitely, to produce large quantities of monoclonal antibodies. For purposes of this invention, the immune lymphoid cells selected for fusion are lymphocytes and their normal differentiated progeny, taken either from lymph node tissue or spleen tissue from immunized animals. Applicants prefer to employ immune spleen cells, since they offer a more concentrated and convenient source of antibody producing cells with respect to the mouse system. The myeloma cells provide the basis for continuous propagation of the fused hybrid. Myeloma cells are tumor cells derived from plasma cells.

It is possible to fuse cells of one species with another. However, it is preferred that the source of immunized antibody producing cells and myeloma be from the same species.

The hybrid cell lines can be maintained in culture in vitro in cell culture media. The cell lines of this invention can be selected and/or maintained in a composition comprising the continuous cell line in the known hypoxanthine-aminopterin-thymidine (HAT) medium. In fact, once the hybridoma cell line is established, it can be maintained on a variety of nutritionally adequate media. Moreover, the hybrid cell lines can be stored and preserved in any number of conventional ways, including freezing and storage under liquid nitrogen. Frozen cell lines can be revived and cultured indefinitely with resumed synthesis and secretion of monoclonal antibody. The secreted antibody is recovered from tissue culture supernatant by conventional methods such as precipitation, ion exchange chromotography, affinity chromatography, or the like.

While the invention is demonstrated using mouse monoclonal antibodies, the invention is not so limited; in fact, human antibodies may be used and may prove to be preferable. Such antibodies can be obtained by using human hybridomas (Cote et al., Monoclonal Antibodies and Cancer Therapy, Alan R. Liss, p. 77 (1985)). In fact, according to the invention, techniques developed for the production of "chimeric antibodies" (Morrison et al., Proc. Natl. Acad. Sci. 81, 6851 (1984); Neuberger et al., Nature 312, 604 (1984); Takeda et al, Nature 314, 452 (1985)) by splicing the genes from a mouse antibody molecule of appropriate antigen specificity together with genes from a human antibody molecule of appropriate biological activity (such as ability to activate human complement and mediate ADCC) can be used; such antibodies are within the scope of this invention.

As another alternative to the cell fusion technique, EBV immortalized B cells are used to produce the monoclonal antibodies of the subject invention. Other methods for producing monoclonal antibodies such as recombinant DNA, are also contemplated.

The immunochemical derivatives of the antibodies of this invention that are of prime importance are immunotoxins (conjugates of the antibody and a cytotoxic moiety). The antibodies are also used to induce lysis through the natural complement process, and to interact with antibody dependent cytotoxic cells normally present.

### Immunotoxins

The cytotoxic moiety of the immunotoxin may be a cytotoxic drug or an enzymatically active toxin of bacterial, fungal, plant or animal origin, or an enzymatically active fragment ("A chain") of such a toxin. Enzymatically active toxins and fragments thereof used are diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolacca americana proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. In another embodiment, the antibodies are conjugated to small molecule anticancer drugs. Conjugates of the monoclonal antibody and such cytotoxic moieties are made using a variety of bifunctional protein coupling agents. Examples of such reagents are SPDP, IT , bifunctional derivatives of imidoesters such a dimethyl adipimidate HCl, active esters such as disuccinimidyl suberate, aldehydes such as glutaraldehyde, bis-azido compounds such as bis (p-azidobenzoyl) hexanediamine, bis-diazonium derivatives such as bis-(p-diazoniumbenzoyl)-ethylenediamine, diisocyanates such as tolylene 2,6-diisocyanate and bis-active fluorine compounds such as 1,5-difluoro-2,4-dinitrobenzene. The lysing portion of a toxin may be joined to the Fab fragment of the antibodies.

Advantageously, monoclonal antibodies specifically binding the domain of the protein which is exposed on the infected cell surface, are conjugated to ricin A chain. Most advantageously the ricin A chain is deglycosylated and produced through recombinant means. An advantageous method of making the ricin immunotoxin is described in Vitetta et al., Science 238, 1098 (1987) hereby incorporated by reference.

When used to kill infected human cells in vitro for diagnostic purposes, the conjugates will typically be added to the cell culture medium at a concentration of at least about 10 nM. The formulation and mode of administration for in vitro use are not critical. Aqueous formulations that are compatible with the culture or perfusion medium will normally be used. Cytotoxicity may be read by conventional techniques.

Cytotoxic radiopharmaceuticals for treating infected cells may be made by conjugating radioactive isotopes (e.g. I, Y, Pr) to the antibodies. Advantageously alpha particle emitting isotopes are used. The term "cytotoxic moiety" as used herein is intended to include such isotopes.

In another embodiment, fusogenic liposomes are filled with a cytotoxic drug and the liposomes are coated with antibodies specifically binding gp160.

### Antibody Dependent Cellular Cytotoxicity

The present invention also makes available a method based on the use of antibodies which are (a) directed against gp160, and (b) belong to a subclass or isotype that is capable of mediating the lysis of HIV virus infected cells to which the antibody molecule binds. More specifically, these antibodies should belong to a subclass or isotype that, upon complexing with cell surface proteins, activates serum complement and/or mediates antibody dependent cellular cytotoxicity (ADCC) by activating effector cells such as natural killer cells or macrophages.

The present invention is also directed to the use of these antibodies, in their native form, for AIDS therapy. For example, IgG2a and IgG3 mouse antibodies which bind HIV-associated cell surface antigens can be used in vivo for AIDS therapy. In fact, since gp160 is present on infected monocytes and T-lymphocytes, the subject antibodies and their therapeutic use have general applicability.

Biological activity of antibodies is known to be determined, to a large extent, by the Fc region of the antibody molecule (Uananue and Benacerraf, Textbook of Immunology, 2nd Edition, Williams & Wilkins, p. 218 (1984)). This includes their ability to activate complement and to mediate antibody-dependent cellular cytotoxicity (ADCC) as effected by leukocytes. Antibodies of different classes and subclasses differ in this respect, and, according to the present invention, antibodies of those classes having the desired biological activity are selected. For example, mouse immunoglobulin of the IgG3 and IgG2a class are capable of activating serum complement upon binding to the target cells which express the cognate antigen.

In general, antibodies of the IgG2a and IgG3 subclass and occasionally IgG1 can mediate ADCC, and antibodies of the IgG3, and IgG2a and IgM subclasses bind and activate serum complement. Complement activation generally requires the binding of at least two IgG molecules in close proximity on the target cell. However, the binding of only one IgM molecule activates serum complement.

The ability of any particular antibody to mediate lysis of the target cell by complement activation and/or ADCC can be assayed. The cells of interest are grown and labeled in vivo; the antibody is added to the cell culture in combination with either serum complement or immune cells which may be activated by the antigen antibody complexes. Cytolysis of the target cells is detected by the release of label from the lysed cells. In fact, antibodies can be screened using the patient's own serum as a source of complement and/or immune cells. The antibody that is capable of activating complement of mediating ADCC in the in vitro test can then be used therapeutically in that particular patient.

Antibodies of virtually any origin can be used according to this embodiment of the present invention provided they bind a gp160 epitope and can activate complement or mediate ADCC. Monoclonal antibodies offer the advantage of a continuous, ample supply. In fact, by immunizing mice with gp160 establishing hybridomas making antibodies to gp160 and selecting hybridomas making antibodies which can lyse infected cells in the presence of human complement, it is possible to rapidly establish a panel of antibodies capable of reacting with and lysing infected cells.

### Therapeutic Uses of the Antibodies

When used in vivo for therapy, the antibodies of the subject invention are administered to the patient in therapeutically effective amounts (i.e. amounts that restore T cell counts). They will normally be administered parenterally. The dose and dosage regimen will depend upon the degree of the infection, the characteristics of the particular immunotoxin (when used), e.g., its therapeutic index, the patient, and the patient's history. Advantageously the immunotoxin is administered continuously over a period of 1-2 weeks, intravenously to treat cells in the vasculature and subcutaneously and intraperitoneally to treat regional lymph nodes. Optionally, the administration is made during the course of adjunct therapy such as combined cycles of tumor necrosis factor and interferon.

For parenteral administration the antibodies will be formulated in a unit dosage injectable form (solution, suspension, emulsion) in association with a pharmaceutically acceptable parenteral vehicle. Such vehicles are inherently nontoxic, and non-therapeutic. Examples of such vehicles are water, saline, Ringer's solution, dextrose solution, and 5% human serum albumin. Nonaqueous vehicles such as fixed oils and ethyl oleate can also be used. Liposomes may be used as carriers. The vehicle may contain minor amounts of additives such as substances that enhance isotonicity and chemical stability, e.g., buffers and preservatives. The antibodies will typically be formulated in such vehicles at concentrations of about 1 mg/ml to 10 mg/ml.

IgM antibodies are advantageous since the antigen is highly specific for the target cells and rarely occurs on normal cells. IgG molecules by being smaller may be more able than IgM molecules to localize to infected cells.

There is evidence that complement activation in vivo leads to a variety of biological effects, including the induction of an inflammatory response and the activation of macrophages (Uananue and Benecerraf, Textbook of Immunology, 2nd Edition, Williams & Wilkins, p. 218 (1984)). The increased vasodilation accompanying inflammation may increase the ability of various anti-AIDS agents to localize in infected cells. Therefore, antigen-antibody combinations of the type specified by this invention can be used therapeutically in many ways. Additionally, purified antigens (Hakomori, Ann. Rev. Immunol. 2, 103 (1984)) or anti-idiotypic antibodies (Nepom et al., Proc. Natl. Acad. Sci. 81, 2864 (1985); Koprowski et al., Proc. Natl. Acad. Sci. 81, 216 (1984)) relating to such antigens could be used to induce an active immune response in human patients. Such a response includes the formation of antibodies capable of activating human complement and mediating ADCC and by such mechanisms cause infected cell destruction.

The antibody of the subject invention also is useful in the diagnosis of HIV in test samples. It is employed as one axis of a sandwich assay for HIV env, together with a polyclonal or monoclonal antibody directed at another sterically-free epitope of HIV env. For use in some embodiments of sandwich assays the 5C2E5 antibody or its equivalent is bound to an insolubilizing support or is labelled with a detectable moiety following conventional procedures used with other monoclonal antibodies. In another embodiment a labelled antibody, e.g. labelled goat anti-murine IgG, capable of binding the 5C2E5 antibody is employed to detect HIV env binding using procedures previously known per se.

Finally, in another embodiment the antibody is immobilized on a water insoluble support and used for the immunoaffinity purification of HIV env, rgp120 or the TCB domain as is described below in greater detail. Any known procedure for immobilizing monoclonal antibodies can be used for this purpose.

In general, the inflammatory or immune-potentiated inflammatory events to be treated with HIV env are characterized by humoral and/or cytotoxic cellular responses directed against foreign or self target tissue which are not desirable in the therapeutic context concerned. Typically, immune-potentiating inflammatory events are characterized by the generation of antibodies and/or a cytotoxic T cell response directed against (1) a host antigen as in the case of undesirable autoimmune diseases or in graft versus host disease, or (2) antigens present in grafted tissue, e.g. organ transplants. Such events are clinically characterized by infiltration of polymorphonuclear neutrophils and mononuclear leukocytes into the target tissue, pain, localized edema, possible vascular endothelial injufry and excessive production of cytokines by stimulated cells.

The HIV env compositions to be used in the therapy will be formulated and dosages established in a fashion consistent with good medical practice taking into account the disorder to be treated, the condition of the individual patient, the site of delivery of the env polypeptide, the method of administration and other factors known to practitioners.

HIV env is prepared for administration by mixing HIV env at the desired degree of purity with physiologically acceptable carriers, i.e., carriers which are nontoxic to recipients at the dosages and concentrations employed. Ordinarily, this will entail combining HIV env with buffers, low molecular weight (less than about 10 residues) polypeptides, proteins, amino acids, carbohydrates including glucose or dextrans, chelating agents such as EDTA, and other excipients. HIV env for use in therapeutic administration must be sterile. This is readily accomplished by sterile filtration through (0.2 micron) membranes. HIV env ordinarily will be stored as lyophilized formulations for reconstitution, but is stable for storage as an aqueous formulation.

Generally, where the disorder permits, one should formulate and dose the HIV env for site-specific delivery. This is convenient in the case of RA and inflammatory bowel disease. In the former case, HIV env is formulated into a sterile sustained release composition suitable for injection into the synovial fluid or implantation into the synovial lining or capsule. In the case of inflammatory bowel disease, the HIV env is formulated into suppositories with pharmaceutically acceptable oleaginous substances as are generally known in the art.

Sustained release formulations will be selected from the classes of microcapsular particles and implantable articles. Liposomal encapsulation is not preferred for injection directly into the synovial cavity in RA because it entails the introduction of lipid into the joint. However, liposomal encapsulation is suitable for implementation of sustained release HIV env into the synovial capsule. For the treatment of RA using sustained-release HIV env, the HIV env is preferably incorporated into a biodegradable matrix or microcapsule. A suitable material for this purpose is a polylactide, although other polymers of poly (α-hydroxycarboxylic acids), such as poly-D-(-)-3-hydroxybutyric acid (EP 133,988A), can be used. Other biodegradable polymers include poly(lactones), poly(acetals), poly(orthoesters) or poly(orthocarbonates). The initial consideration here must be that the carrier itself, or its degradation products, is nontoxic in the target tissue and will not further aggravate the disease. This can be determined by routine screening in animal models of the target disorder or, if such models are unavailable, in normal animals. For examples of sustained release compositions, see U.S. patent 3,773,919, EP 58,481A, U.S. patent 3,887,699, EP 158,277A, Canadian patent 1176565, U. Sidman et al., "Biopolymers" 22:547 [1983], and R. Langer et al., "Chem. Tech." 12:98 [1982].

The dosage of HIV env to be employed is dependent upon numerous factors, including the T4 binding affinity of the HIV env preparation, the half-life of the env preparation, the route of administration, the clinical condition of the patient (including the presence of infections), the presence of anti-HIV env antibodies, and whether or not the HIV env is to be used for the prophylaxis or for the treatment of acute autoimmune or transplant rejection episodes. As a general proposition, the HIV env dose delivered at the target site should be about from 0.5 x 10⁻⁸M to 5 x 10⁻⁹M. Typically, the HIV env concentrations in the therapeutic dosage forms should be delivered by continuous infusion, sustained release of injection at empirically determined frequencies. Since RA synovial fluid can be present in amounts up to 50 ml, for example in a knee joint, the actual amount of HIV env will depend upon its dilution into the synovial fluid as well as the empirically determined losses of HIV env to endogenous proteases, leakage into the general circulation and sequestration by any anti-HIV env that is present. Thus it is best to evaluate the efficacy of the initial dosage protocol by withdrawing synovial fluid samples for HIV env T4 binding assay during early stages of treatment in order to determine the proper dosage regimen.

The HIV env therapy may be delivered in concert with other anti-inflammatory substances such as salicylate, nonsteroidal anti-inflammatory drugs, penicillamine, gold salts, TGF-β, TNF-α or TNF-β antagonists (described in copending U.S.S.N. 898,272), γ-interferon antagonists and/or IL-1 antagonists. It is not necessary that such substances be included in the HIV env compositions per se, but the anti-inflammatory substances can be formulated together with HIV env.

HIV env concentrations localized at the sites of inflammation may exceed the whole body maximum therapeutic dose. Assay of the HIV env concentration in inflammatory infiltrates will provide guidance as to the amount of HIV env to be intravenously injected, particularly if localized administration is impractical. The key factor in selecting an appropriate dose is the result obtained. If the patient's inflammatory response does not at least partially resolve within about 48 hours after administration, the dose should be gradually elevated until the desired effect is achieved. Correspondingly higher doses of HIV env preparations having lower T4 binding capacity and/or lower affinity for T4 will be required, and vice versa for preparations with greater capacity and affinity. Also, relatively higher doses initially will be needed for the treatment for acute rejection or inflammatory episodes, i.e., for patients in acute organ transplant rejection or undergoing arthritic flares.

In view of the therapeutic urgency attendant acute rejection episodes, HIV env should be intravenously infused or introduced at the inflammatory lesion immediately upon the development of symptons or serological evidence of rejection. On the other hand, prophylaxis is suitable accomplished by intramuscular or subcutaneous administration.

The invention will be more fully understood by reference to the following examples. They should not, however, be construed as limiting the scope of the invention. All literature citations are incorporated by reference.

### EXAMPLE 1

It has been shown that the initial event in T-helper infection with HIV involves the selective interaction between HIV envelope glycoproteins and the T4 surface structure (1-6,15,23-26). More specifically, McDougal et al, showed that HIV can selectively bind to the T4A epitope of the T4 antigen complex (25). Further, much of the impaired immunologic functions in patients infected with AIDS virus have been linked to the selective ability of HIV to infect and eventually destroy the helper cell population (1,2,9,10). It has also been suggested that PBMC from AIDS patients are hyporesponsive to mitogenic and antigenic, particularly tetanus toxoid, stimulation (12) possibly due to HIV associated depletion of helper cell function. This Example was directed principally at determining the activity of rgp120 on the tetanus toxoid response in normal PBMC.

### Materials and Methods

Recombinant HIV envelope glycoprotein (rgp120): Chinese hamster ovary cells transfected with an HIV envelope gene expression plasmid (20) were used for the production of the rgp120 used in these samples. The rgp120 was purified from the conditioned cell culture medium by immunoaffinity chromatography and was >98% pure as determined by SDS-polyacrylamide electrophoresis.

Isolation of peripheral blood mononuclear cells (PBMC): Heparinized syringes were used for the collection of blood from healthy donors. Each blood sample was diluted with an equal volume of saline, layered on a Ficoll-Hypaque gradient (sp.gr.1.08) and centrigued at 400xg for 40 min. at room temperature. PBMC at the plasma-Ficoll interface were removed, washed three times in Hank's balanced salt solution (HBSS) (Gibco; Grand Island Biological Co, Grand Island, NY), resuspended in complete medium and counted. Cell viability as determined by trypan blue exclusion was consistently greater than 95%.

Culture medium: RPMI 1640 medium purchased from Gibco was used. It was supplemented with 10% heat inactivated fetal bovine serum (Hyclone, Logan, Utah), 2mM L-glutamine, 10 mM HEPES, penicillin/streptomycin (Gibco), and 5 x 10⁻⁵. M2-mercapteothanol (Sigma, St. Lous, MO). The same medium, without 2-mercaptoethanol was used for polyclonal activation. RPMI 1640 with 2% heat inactivated fetal bovine serum was used for staining of cells with monoclonal antibodies.

Tetanus toxoid (TT) induced proliferative activities: The RPMI 1640 medium used for this assay was supplemented with 10% autologous plasma, 2 mM L-glutamine and antibiotics. PBMC were cultured in quadruplicate as 10⁵ cells in 200 µl medium per well in a microtiter plate in the presence or absence of TT (obtained from Dr. A Muchmore, National Cancer Institute, Bethesda, MD) at a final dilution of 1:300 as established by preliminary experiments for attainment of optimal response. Replicate cultures containing various concentrations of rgp120 were established in parallel. After six days of incubation when peak responses occurred, cultures were pulsed with [³H]-thymidine, processed and counted as described above.

Staining of cells with monoclonal antibodies: Fluorescein isothiocyanate (FITC) conjugated monoclonal antibodies (MAb) OKT3, OKT4, OKT4A, and OKT8 were purchased from Ortho Diagnostic Systems, Inc., (Raritan, N.J.). Aliquots of cells (10⁶/50µl) were mixed with FITC-conjugated OKT3, OKT4, OKT4A, OKT8 or the murine IgG2ab control (5µl). After 40 min. of incubation on ice, the cells were washed twice in media, fixed in 2% paraformaldehyde and samples were analysed using a fluorescein activated cell sorter (FACS IV, Becton Dickinson, Mt. View CA) equipped with a 4 watt argon laser at a wavelength of 488 nm. Ten thousand cells were aquired by list mode and measurements were performed on a single cell basis and were displayed as frequency distribution histograms (21). Dead cells and debris were gated out of the analysis on the basis of forward light scatter. The data are expressed as percent specifically stained cells (number of fluroescein labeled cells/number of cells analyzed x 100) less non specific staining (IgG2ab-FITC control). The mean channel of intensity of stained cells was also determined but is not presented.

Statistical analysis: The data were analyzed by the paired design Student's test and by analysis of variance for multiple group comparison.

### Results

The influence of rgp120 on PBMC response to a soluble specific antigen was investigated. Six experiments were performed in which TT was used as the stimulus with and without rgp120 added to the cultures. The results are presented in Table 2, demonstrating that rgp120 can cause significant inhibition (p=0.05) of PBMC proliferative response to a specific antigen in normal PBMC. The extent of inhibition varied in these five experiments ranging from 19 to 51 percent of control values. No inhibition was observed in experiment No. 6 (not shown).

**Table 2**

| rgp120 inhibition of TT-induced PBMC proliferative response | | | |
|---|---|---|---|
| Exp. No. | [³H]-Thymidine Incorporated, cpm x 10-³* | | |
| | Control | +rgp120 | % inhibition |
| 1 | 199 | 145 | 27 |
| 2 | 144 | 115 | 21 |
| 3 | 60 | 49 | 19 |
| 4 | 90 | 44 | 51 |
| 5 | 52 | 23 | 43 |

| | | | |
|---|---|---|---|
| *Quadruplicate cultures of PBMC were established in flat bottom microtiter plates at 10⁵ cells/200 µl/well in the presence of TT only (control) or TT plus 5 µg rpg120/ml (+rgp120). After 6 days of incubation the cultures were pulsed with [³H]=thymidine, harvested, processed, and counted. Each data point represents the mean cpm and the percent inhibition was calculated as follows. 1 - (cpm in the presence of rgp120/control cpm) x 100. p<0.05. | | | |

Interaction of rgp120 with various T-cell subsets: It has been suggested that infection of T-cells by HIV is initiated by the selective interaction of the virus envelope glycoproteins with the T4 component of the T-helper membrane receptor complex (1-6, 15,23-26). Therefore, it was of interest to determine whether rgp120 has the same selective ability of binding to human lymphoid cells. PBMC were incubated in the absence or presence of rgp120 (5µg/ml) for 24 hr at 4°C and at 37°C. The cells were washed and stained directly with FITC-conjugated OKT4 and OKT4A. The results of FACS analysis show that the treatment of cells with rgp120 at 4°C did not interfere with the binding of OKT4. The treatment with rgp120 at 37°, however, caused an alteration in OKT4 binding as reflected by a reduction in fluorescein intensity of T4+ cells. Conversely, the binding of OKT4A was completely absent following incubation of cells with rgp120 at 37°C. These results indicate that similar to HIV (25,26) rgp120 can occupy the T4A epitope or a neighboring epitope of the T4 helper membrane receptor.

The selective binding of rgp120 to the T4A epitope was examined further in a series of experiments involving the treatment of cells with a high concentration of 100 µg rgp120 per ml for 24 hr at 37°C. The cells were then washed and stained with FITC-conjugated OKT3, OKT4, OKT4A, and OKT8. The results of FACS analysis showed that the pretreatment of PBMC with rgp120 completely blocked the T4A epitope of T-helper membrane surface antigen. The binding of OKT3 and OKT8 ro rgp120 treated cells was similar to that of the untreated cell preparation indicating that even at high concentrations, rgp120 does not bind to the T3 or the T8 membrane surface antigen. It is interesting to note that rgp120 treatment did not completely block the T4 receptor but only diminished the mean channel of the fluorescence of T4 stained cells. This may indicate a partial binding and/or and alteration in membranal structures of the T-helper receptor.

### EXAMPLE 2

### Anti-TCB Domain Monoclonal Anitbody

This example describes the procedure by which monoclonal antibody 5C2E5 was obtained. A female Balb/c mouse was immunized with about 30 micrograms rgp120 per dose over a period extending approximately seven months. An initial 3-site subcutaneous vaccination with rgp120 in complete Freund's adjuvant was followed by 4 3-site subcutaneous injections in incomplete Freund's adjuvant and then by 2 3-site subcutaneous injections in phosphate buffered saline. A final intraperitoneal boost also in phosphate buffered saline.

The hybridoma that secreted monoclonal antibody 5C2E5 was obtained by the general method of Oi et al. (37) using the mouse myeloma cell line NP₃x63-Ag8.653 and spleen cells from the rgp120 immunized animal described above. This cell line is widely available to the public. The fused cell culture was plated out into 10 plates, each of which contained 60 wells per plate. Approximately 480 wells (80%) contained viable cells. Each well was screened for antibody against rgp120 by radioimmunoprecipitation or ELISA. The ELISA was a conventional double antibody sandwich assay using (a) rgp120 coated microtiter wells in which nonspecific binding sites had been blocked by incubating the coated wells for 30 min. at room temperature with 1% (wt/vol) and (b) horseradish peroxidase labelled goat anti-mouse IgG. 10 wells out of 480 were positive for antibody to rgp120. These 10 wells were then screened for the ability to block the binding of rgp120 to human T4 helper cells. The blocking assay was conducted as follows:

Ascites fluid, in vitro culture fuid supernatant or purified preparations of ascites or supernatant are screened in the typical assay. The purification method, for example, comprises adsorption to a column of insolubilized staphylococcal protein A followed by elution of the antibody. Serial dilutions of the monoclonal antibody preparation, from undiluted to 1:1000, were prepared in assay buffer. Assay buffer contained the following components:
a) Gibco F-12/DMEM 50:50 mix (media).
b) 10% Extensively dialyzed fetal bovine serum.
c) .2mM Phenylmethylsulfonyl Fluoride.
d) .05% Tween 80 in phosphate buffered saline.
e) .06M NaCl.
f) .25 mg/ml BSA (bovine serum albumin).
g) 12.5 mM Hepes Buffer pH 7.4.

50 µl of each serial dilution was combined in 12 x 75 polystyrene test tubes with 50-100 µl of radioiodinated rgp120 (^{∼}100,000 cpm) in assay buffer and 100 µl of assay buffer containing 3-5x10⁵ CHO (chinese hamster ovary) cell line SVE OKT4 (clone 17) and incubated for 1 hour at 4°C. The cells were centrifuged to pelletize the cells, the supernatant aspirated, the pellet resuspended in 1.0 ml of assay buffer, repelletized, aspirated and the pellet counted in a gamma counter.

The CHO cell line was a transformant CHO cell line in which the gene encoding the human T4 receptor was placed in a DHFR-encoding vector under the control of the SV-40 early promoter, transfected into CHO cells and amplified in 500 nM methotrexate in order to amplify the T4 receptor gene and increase its expression on the transformant cell surface. Other T4 transformants that are suitable for use in this assay are known in the art and readily produced from publicly available starting materials (38,39,40). Preferably, such T4 transformants are cotransformed with DHFR and amplified by conventional techniques in order to maximize T4 cell surface expression. Such cells, containing large populations of T4, provide greater sensitivity to the assay.

It is important to use care, in radioiodinating rgp120. Several otherwise conventional methods were found to result in ¹²⁵I-rgp120 havng binding characteristics that rendered the tracer unusable in the assay. The sucessful method employed was as follows: 400µl of rgp120 (1mg/400µl) was mixed with 1 mCi ¹²⁵I, 20µl of lactoperoxidase (0.35mg/ml) and 20µl of 1mM H₂O₂, incubated for 10 min. at room temperature, 20µl of beta-mercaptoethanol (1mM) added, the reaction mixture incubated for 2 min. at room temperature, 50µl of 0.5% wt/vol BSA added, the reaction mixture diluted to 2.5 ml in PBS buffer containing 0.5 wt/vol BSA (PBS-BSA) loaded on a G-25 column and eluted with 3.5ml of PBS-BSA. ¹²⁵I-rgp120 was recovered in the eluate.

The cells in the sole blocking antibody-producing well (5C₂) were subcloned. The same inhibitory activity was obtained from well E5. This activity, attributed to IgG, was purified as desired by ammonium sulfate precipitation or the like. The 5C2E5 monoclonal antibody exerted maximal inhibition of ¹²⁵I-rgp120 T4 receptor binding by reducing bound counts to about 10-15% of murine monoclonal antibody controls as measured by the reduction in pellet cpm. The 5G2E5 hybridoma is available to the public (ATCC HB9435).

### EXAMPLE 3

### Recovery of TCB Domain from rgp120

This example describes the procedure used to prepare the portion of TCB domain spanning residues 411-454.

The rgp120 used in this example was purified from the culture fluid of CHO cells transfected with pAIDSenvTrDHFR and cultured as described in EP 187,041. The rgp120 was purified to near homogeneity by immunoaffinity chromatography. The purified rgp120 was hydrolyzed in 0.25 M glacial acetic acid, under a reduced oxygen atmosphere, at 110°C for 18 hours according to the procedure of Ingram (35). Under these conditions the rgp120 protein backbone is cleaved on both the N and C terminal sides of aspartyl residues. The digested rgp120 was neutralized by the addition of Tris buffer and adjustment of the pH to 7.

The digested rgp120 was then passed over a column of murine monoclonal antibody 5C2E5 covalently coupled to aldehyde-silica by a standard procedure (36). The column was then washed with a saline solution buffered at pH 3. Monoclonal antibody 5C2E5 has been shown to bind to rgp120 in such a manner as to prevent the binding of rgp120 to the T4 surface antigen of T4+ helper T cells (see example 2). The eluent from the 5C2E5 column was analyzed by a standard method of quantitative amino acid analysis and the following amino acid composition was obtained:

| amino acid | D/N | T | S | E/Q | P | G | A | C | V | N | I | L |
|---|---|---|---|---|---|---|---|---|---|---|---|---|
| moles/mole rgp120 | 3.0 | 3.9 | 2.8 | 4.2 | 3.4 | 3.1 | 1.9 | 0.8 | 1.4 | 1.5 | 5.2 | 3.8 |

| amino acid | Y | F | K | R | | | | | | | | |
|---|---|---|---|---|---|---|---|---|---|---|---|---|
| moles/mole rgp120 | 1.0 | 1.6 | 2.1 | 2.6 | | | | | | | | |

The conditions under which the analysis was performed preclude the accurate determination of C and W. The eluent from the 5C2E5 column was also analyzed by N-terminal sequencing and only the following sequence was obtained: _ I _ L P _ R I K Q F.

The amino acid analysis and N-terminal sequencing data confirm that the only peptide in the acetic acid digest of rgp120 that bound to the 5C2E5 column was the 44 amino acid fragment from threonyl residue 411 to arginine residue 454 of the HIV env sequence. This region has the following sequence: TITLPCRIKQFINMW-QEVGKAMYAPPISGQIRCSSNITGLLLTR. This peptide contains the sequence deleted in the delta 3 mutant described above and the epitope of the murine monoclonal antibody 5C2E5 which blocks the binding of rgp120 to the T4 surface antigen of T4+ helper T cells, thereby inferring that this peptide is a portion of the TCB domain.

### EXAMPLE 4

### Immunotoxins

Monoclonal antibodies directed against HIV-encoded proteins which (either separately or as a cocktail) are coupled to deglycosylated ricin A chain (dgA) and are used to kill cells infected with HIV from a wide variety of viral subtypes, are generated as follows:

Sera are obtained from mice immunized (multiple i.p. injections) with heated (30ʹ/60°) NP-40 lysates from HIV-infected human Hp (CD4⁺) cells. Sera can also be obtained from mice immunized with gp120 or gp150. The protein gp150 is the extracellular domain of gp160 with the region which clips to form gp120 and gp41, removed. All sera are tested by:
1. The indirect immunotoxin (IT) assay using either HIV infected H9 cells, uninfected H9 cells, or gp120-coated unifected H9 cells.
2. Radioimmunoassay (RIA) - Positive sera are tested by RIA against purified gp120 and gp150 to determine if the mice have made antibodies against these proteins.

Sera from mice which are positive in one or both of the RIAs and the indirect IT assay are collected, pooled and affinity-purified on Sepharose-gp120 or Sepharose-gp150. The purified antibody is again tested in the three assays. If positive, the mice are boosted with lysates of HIV-H9, ±gp150 ±gp120. Several mice are sacrificed and their spleens fused with Sp2/0 or NP3X63-. Ag8.653 myeloma cells. Supernatants (SN) from wells containing growing hybridomas are tested in the indirect IT assay against (a) H9 vs. (b) HIV-H9. Those hybridomas that are positive in (b) and negative in (a) are further subcloned and tested by RIA, indirect IT, immunoprecipitation, et. Antibodies from positive clones are isotyped and the production of antibody scaled up. The antibodies are coupled directly to dgA. These IT-dgAs are tested on HIV-infected cells and cells from AIDS patients. ITs for clinical use are then prepared.

### Ricin A-chain Antibody Conjugate

### Preparation of F(abʹ)₂ and FABʹ Fragments of Antibody

The antibody preparations are treated with pepsin (4500 U/ml) (Sigma, St. Lous, MO) for 6 hours at 37°C under the following conditions: pH = 3.7 (0.1 M citrate buffer); protein concentration, 2-3 mg/ml; enzym/protein ratio, 2/100 by weight. The digestion is terminated by raising the pH to 8.0 with 1 N NaOH. F(abʹ)₂ fragments are isolated by gel filtration on Sephacryl S-200HR (Pharmacia, Piscataway, NJ) equilibrated in phosphate-buffered 0.3 M NaCl or by adsorbing the non-neutralized digest to a column (10 x 2 cm) of SP-Sephadex equilibrated in 0.1 M citrate buffer, pH 3.7, and eluting the F(abʹ)₂ fragment with phosphate-buffered saline (PBS), pH 7.2. The yields of F(abʹ)₂ fragments generally range from 35-50%.

The Fabʹ fragment is obtained by reducing the F(abʹ)₂ fragment (5-10 mg/ml) with dithiothreitol (DTT) at a final concentration of 5 mM in 0.1 M phosphate-buffer, pH 7.5 containing 0.003 M ethylenediaminetetraacetic acid (disodium salt) (EDTA) (PBE) for 1 hour at room temperature. The excess DTT is removed by gel filtration on Sephadex G-25 and the thiol groups of the Fabʹ fragment (5 mg/ml) are derivatized with Ellman's reagent (5,5ʹ-dithio-bis(2-nitrobenzoic acid) (DTNB) at a final concentration of 2 mM, as described in Fulton et al., J. Immunol., 136: 3103-3109 (1986) hereby incorporated by reference. The nonreacted DNTB is removed by gel filtration on a column (30 x 2 cm) of Sephadex G-25 equilibrated in PBE.

The purity of the F(abʹ)₂ and Fabʹ fragments is determined by SDS-PAGE and by double diffusion and immunoelectrophoresis using anti-mouse IgG sera which reacts with both Fabʹ and Fc fragments. The preparations are free of both Fc fragments and intact IgG.

### Ricin A. Chain

Deglycosylated A chain (dgA) is prepared as described by Thorpe et al., Eur. J. Biochem., 147: 197-206 (1985) hereby incorporated by reference. The LD50s of native and dgA chain in 25 g mice are about 0.7 mg and 0.3 mg, respectively. The IC₅₀ in a cell-free rabbit reticulocyte assay is about 10⁻¹¹-10⁻¹² M for both dgA and native A chain.

For conjugation with the antibody, the A chain is reduced with 5 mM DDT as described in Fulton et al. supra.

### Preparation of IT-As with SPDP

IT-As are prepared using IgG or F(abʹ)₂ fragments of antibodies as described in Fulton et al. supra. Briefly, SPDP dissolved in dimethylformamide is added to a solution of IgG or F(abʹ)₂ (10 mg/ml) in PBE, pH 7.5, to give a final concentration of 1 mM. After 30 minutes at room temperature, the solution is filtered on a column of Sephadex G-25 (30 x 2 cm) equilibrated with PBE. The degree of substitution of derivatized IgG and F(abʹ)₂ fragment is about 3-4 molecules PDP/molecule of protein. The derivatized protein is then mixed with reduced A chain (dissolved in PBE) using 1.3 mg/ml and maintained for 2 hours at 25°C and overnight at 4°C. The mixture is then purified.

### Preparation of FabʹAs with DTNB

The preparation of mouse Facʹ-A with DTNB-derivatized Fabʹ is performed as described for rabbit Fabʹ-A in Fulton et al. supra. Briefly, the Ellman's-derivatized Fabʹ fragment containing 1-2 TNB-substituted thiol groups (see preparation of Fabʹ) dissolved in PBE (5 mg/ml) is mixed at room temperature with reduced A chain using 1.3 mg A chain/mg Fabʹ at a final concentration of 2 mg protein/ml. The reaction between TNB-Fabʹ and A chain is followed by the increase of absorbance at 412 Nm and is complete after about 2 hours at 25°C. The mixture is then immediately purified.

### Purification of the IT-As

The IT-As prepared with intact antibodies or their fragments are purified by affinity chromatography on Blue Sepharose using a modified version of the method described by Knowles and Thorpe Anal. Biochem. 160: 440-443 (1987) hereby incorporated by reference. Chromatography is carried out in 0.05 M phosphate buffer, pH 7.0 and A chain and IT-As are eluted with 1 M NaCl prepared in the same buffer. The eluate is concentrated by ultrafiltration to 5mg/ml and applied to Sephacryl S-200HR equilibrated with phosphate-buffered 0.3 M NaCl, pH 7.2. The peak(s) containing the purified IT-A is collected, concentrated by ultrafiltration to at least 0.5mg/ml, and stored in aliquots at -70°C.

### Preparation of Fabʹ-GAMIg-A and the Indirect Immunotoxin Assay

Fab fragments of affinity-purified goat anti-mouse immunoglobulin (GAMIg) are prepared by papain digestion using 2% papain in 0.1 M NaPO₄, pH 7.0 containing 0.1 M EDTA and cysteine for 5 hours at 37°C. Fab fragments are separated from Fc fragments by affinity chromatography on SEAE-Sephacel equilibrated with 0.01 M NaPO₄, pH 7.6. Fab fragments are desalted on Sephadex G-25. Reduced Fab fragments are then reacted with a 100-fold molar excess of 5,5ʹdithio-bis(2-nitrobenzoic acid) (DTNB, Ellman's reagent). Fab-GaMIg substituted with Ellman's reagent (E) and are separated from free Fab-GAMIg and free Ellman's reagent by chromatography on S200.

Ricin A chain prepared and tested according to Fulton et al. (J. Biol. Chem. 261, 5314 (1986) is reduced with 5 mM DTT. Reduced A chain is desalted on Sephadex-G25 and A chain coupled to the Fab-GAMIg-E. Fab-GAMIg-A is then purified by affinity chromatography on both S200 and Blue Sepharose.

### Indirect Immunotoxin Assay

Cells are used in logarithmic growth phase when cell viabilities are greater than 95%. Serial dilutions of primary unconjugated antibody ranging in concentration from 2 x 10⁻⁸ to 2 x 10⁻¹³ M are prepared in the appropriate media and 100 µl aliquots are plated in triplicate in 96 well plates. 10⁵ H9 to HIV-H9 cells in a volume of 100 µl of the same medium are added to each well and plates are incubated for 1 hour at 4°C. Fab-GAMIg-A (final concentration 1µg/ml) is then added to the appropriate wells. Controls included: 1) untreated cells; 2) cells treated with Fab-GAMIg-A only; 3) cells treated with antibody only; 4) cells treated with antibody followed by Fab-GAMIg (no A chain). The plates are then incubated for 36 hours at 37°C in 5% CO₂. Cells are pulsed for 4-8 hours at 37°C with 5 µCi of ³H-thymidine and then harvested on glass fiber filters using a Titertek automatic harvester. Incorporation of ³H-thymidine is determined by liquid scintillation counting in a LKB Beta Counter. Results are expressed as a percentage of the ³H-thymidine incorporated by the untreated cells. Cells in all the control cultures incorporate 80-100% of the ³H-thymidine taken up by untreated cells.

### Data Analysis

The predicted potency of a monoclonal antibody in the indirect IT assay is based on the concentration of antibody (used with an optimal concentrationof Fab-GAMIg-A) that will kill 50% of target cells (this is the IC₅₀). For IT-As to be useful in vivo, the IC₅₀ should be 10⁻¹² - 10⁻¹⁰ M.

IC₅₀'s are ranked as follows:
10⁻¹¹ M = 4+
10⁻¹⁰ M = 3+
10⁻⁹ M = 2+
10⁻⁸ M = 1+
4⁺ and 3⁺ antibodies are considered to be good condidates for IT-As and are further tested by RIA against gp150 and gp120.

### RIA

Wells of 96 well microtiter plates are coated with 5 µl/ml gp120 or gp150 in 100µl PBS for 16 hours/4°C. Wells are decanted, washed 5 times in dH₂O and blocked with 200 µl PBS-10% FCS for 24 hours/4°C. Wells are decanted and washed 5 times in dH₂O. Dilutions of sera or supernatant (SN) are added for 4 hours at 25°C. Material is removed and wells are washed 5 times in dH₂O. 10⁵ cpm GAMIg is added for 4-6 hours/25°C. Samples are removed, plates are washed 5 times in dH₂O, dried, cut and counted.

### Antibodies and Sera Tested

A) Rabit anti-gp41.
B) MoAbs Nos. 1,2,3,4,5,6,7,8,10,12 (coded).
C) MoAbs 7F11, 1F9, 5B9, 1D10, 5G9, 5C2, 6D8E9, 9F6 (coded).
D) Polycolnal mouse serum generated by immunizing BALB/c mice with lysates from HIV-H9 cells.

### Results

### Monoclonal Antibodies

1. Rαgp41 : IC₅₀ 1 x 10⁻⁸ M (non-affinity purified):-
2. Monoclonal Antibodies.
   7F11 : +1
   1F9 : -
   5B9 : -
   1D10 : -
   5G9 : -
   5C2 : -
   6D8E9 : -
   9F6 : -

Only one monoclonal antibody was modestly effective in the indirect assay.

Initially, 50 BALB/c mice were immunized with a lysate prepared from a x 10⁸ H9 cells which were infected with HIV-I. The sera from these mice were screened in the indirect assay. Fifteen of the surviving 38 mice were 3⁺-4⁺ in the indirect IT assay. RIAs were done and after two immunizations, 3 mice had developed significant levels of antibody to gp120 (see Table 3).

**TABLE 3**

| **RESULTS OF THE INDIRECT IT ASSAY AND THE RIA** | | | |
|---|---|---|---|
| **Indirect IT** | | **RIA** | |
| Mouse | IC₅₀ (after 2 boosts) | µG/ML anti-gp120 (after 5 boosts) | anti-gp15 |
| 302 | 4+ | 2+ | |
| 304 | 1+ | 4+ | |
| 306 | 1+ | 4+ | |
| 310 | - | 3+ | |
| 312 | 1+ | 3+ | |
| 314 | 3+ | 4+ | |
| 316 | 1+ | 2+ | |
| 318 | 1+ | 3+ | |
| 320 | 1+ | 3+ | |
| 332 | - | 2+ | |
| 334 | 2+ | 2+ | |
| 336 | 4+ | 4+ | |
| 338 | 1+ | 2+ | |
| 340 | 1+ | - | |
| 344 | 3+ | 2+ | |
| 348 | 2+ | 4+ | |
| 350 | 4+ | 3+ | |
| 352 | 2+ | - | |
| 354 | 2+ | - | 2+ |
| 356 | 1+ | 2+ | - |
| 358 | 3+ | 3+ | - |
| 362 | 4+ | 4+ | 4+ |
| 364 | 4+ | 3+ | 3+ |
| 366 | 2+ | 3+ | 4+ |
| 368 | 2+ | 4+ | 4+ |
| 370 | 4+ | 4+ | 4+ |
| 372 | - | 1+ | - |
| 374 | - | 3+ | - |
| 376 | 4+ | 3+ | 4+ |
| Values of 1+ or greater were greater than 1 µg/ml. | | | |

### Bibliography

1. Barre-Sinoussi, F.et al., Science 220: 868 (1983).
2. Popovic, M. et al., Science 224: 497 (1984).
3. Gallo, R.C. et al., Science 224; 500 (1984).
4. Schupbach, J. et al., Science 224; 503 (1984).
5. Sarngadharan, M.G. et al., Science 224: 506 (1984).
6. Fauci, A.S. et al., Annals. Intern. Medicine, 102: 800 (1985).
7. Lane, H.C. et al., N. Engl. J. Med. 309: 453 (1983).
8. Murray, H. W. et al., N. Engl. J. Med. 310: 883 (1984).
9. Bowen, D.L. et al., Annals Intern. Med. 103: 704 (1985).
10. Fauci, A.S., Clin. Res. 32:491 (1984).
11. Rook, A.H. et al., J. Clin. Invest. 72: 398 (1983).
12. Lane, H.C. et al., N, Engl. J. Med. 313: 79 (1985).
13. Ammann, A.J. et al., Clin. Immunol. Immunopathol. 27: 315 (1983).
14. Stahl, R.E. et al., Am. J. Med. 73: 171 (1982).
15. Vilmer, E. et al., Lancet 1: 753 (1984).
16. Koenig, S. et al., Science 233: 1089 (1986).
17. Levy, J.A. et al., Virology 147: 441 (1985).
18. Seigel, J.P. et al., J. Clin Invest. 75: 1957 (1985).
19. Cunningham-Rundles, S. et al., J. Clin. Immunol. 3: 156 (1983).
20. Lasky, L.A. et al., Science 233: 209 (1986).
21. Steinkamp, J.A. et al., Science 215: 64 (1982).
22. No reference.
23. Klatzmann, D. et al., Nature 312: 767 (1984).
24. Dalgeish, A.G. et al., Nature 312: 763 (1984).
25. McDougal, J.S. et al., Science 231: 382 (1986).
26. McDougal, J.S. et al., J. Immunol. 135: 3151 (1985).
27. No reference.
28. No reference.
29. No reference.
30. No reference.
31. No reference.
32. Boulaimne, G. et al., Nature, 312: 643 (1984).
33. Perez, P. et al., Nature 316: 354 (1985).
34. Modrow, S. et al., J. Virology 61(2): 570 (1987).
35. Ingram, V.M. Methods in Enzymology Vol. VI: 831-848 (1963).
36. Roy, S.K., et al., Journal of Chromatography, 303: 225-228 (1984).
37. Oi, V. et al. In: Selected Methods in Cellular Immunology, B. Mishell et al. (Eds.), W.J. Freeman Co., San Francisco, CA, p. 351, (1980).
38. Maddon, P. et al., Cell 47: 333-348 (1986).
39. Dalgleish, A. et al., Abstract, International Conference on AIDS, Paris (1986).
40. Tersmette, A. et al., Abstract, International Conference on AIDS, Paris (1986).

## Claims (Claims for the following Contracting State(s): DE, GB, FR, IT, NL, SE, CH, AT, BE, LU, LI)

1. Use of HIV env comprising the TCB domain in the manufacture of a medicament for the treatment of immunoinflammatory episodes.

2. Use according to claim 1 wherein the episode is associated with inflammatory bowel disease, rheumatoid arthritis, systemic lupus erythymatosis, juvenile-onset diabetes, Graves disease, myasthenia gravis, graft-versus-host rejection, and host-versus-graft rejection.

3. Use according to claim 1 or claim 2 wherein HIV env is rgp120.

4. Use according to any one of the preceding claims wherein the medicament is for administration in a therapeutic dose of about from 0.5 x 10⁻⁸ to 5 x 10⁻⁹ molar.

5. Use according to any one of the preceding claims wherein the medicament is a sustained release composition.

6. Use according to claim 5 wherein the sustained release composition is a polylactide.

7. Use according to any one of claims 1-4 wherein the medicament is for intravenous injection.

8. A composition comprising the TCB domain of HIV env which is free of HIV immune epitopes ordinarily flanking the C-terminal or N-terminal ends of the TCB domain.

9. A composition comprising the TCB domain of HIV env which is free of HIV env sequence flanking the C-terminus or N-terminus of the TCB domain.

10. The composition of claim 9 wherein the TCB domain has the amino acid sequence

TEGSNNTEGSDTITLPCRIKQFINMWQEVGKAMYAPPISGQIRCSSNITGLLLTRD
GGNNNNESEI.

11. The composition of claim 9 wherein the TCB domain has the sequence CRIKQFINMWQEVGKAMYAPPISGQIRC.

12. The composition of any one of claims 8-11 which is sterile and which further contains a pharmacologically acceptable vehicle.

13. The composition of any one of claims 8-12 for pharmaceutical use.

14. Use of the composition of any one of claims 8-13 in the manufacture of a medicament for the treatment of inflammatory episodes.

15. Use of a composition of any one of claims 8-13 in the manufacture of a medicament for the treatment of AIDS.

16. Use according to claim 15 wherein the medicament is a vaccine and includes an adjuvant.

17. Use according to claim 15 or claim 16 wherein the composition includes a tumor necrosis factor or an interferon.

18. Use according to claim 17 wherein the tumor necrosis factor is tumor necrosis factor-α and the interferon is interferon-γ.

19. A monoclonal antibody which is capable of blocking or binding the TCB domain.

20. The antibody of claim 19 which binds to the TCB domain as defined in claim 10 or 11.

21. The antibody of claim 19 or 20 which is conjugated to a cytotoxin.

22. The antibody of claim 21 wherein the cytotoxin is ricin.

23. The antibody of claim 19 or 20 which is covalently bound to a detectable marker or a water insoluble matrix.

24. The antibody of any one of claims 19-23 in a sterile pharmaceutically acceptable vehicle.

25. An antibody according to any one of claims 19-24 for the treatment of HIV infection.

26. Use of the antibody of claim 19 or 20 in the manufacture of a medicament for the treatment of HIV infection.

27. A conjugate of (a) a substance capable of binding to a cell surface marker of a cell population against which a cytotoxic T cell cytolytic response is desired and (b) the TCB domain of HIV env which is free of HIV immune epitopes ordinarily flanking the C-terminal or N-terminal ends of the TCB domain which is capable of binding to the T4 receptor of helper lymphocytes.

28. The conjugate of claim 27 wherein the cell surface marker is a protein.

29. The conjugate of claim 27 or claim 28 wherein the conjugate comprises an HIV env fragment having the sequence

TEGSNNTEGSDTITLPCRIKQFINMWQEVGKAMYAPPISGQIRCSSNITGLLLTRD
GGNNNNESEI.

30. The conjugate of any one of claims 27-29 wherein the substance capable of binding the cell surface marker comprises the variable region of an antibody.

31. The conjugate of claim 30 wherein the antibody is capable of binding TGF-α.

32. The conjugate of claim 27 wherein the marker binding substance is a hapten.

33. The conjugate of any one of claims 27-32 wherein the substance capable of binding to the cell surface marker and the TCB domain are cross-linked by a covalent bond.

34. The conjugate of claim 33 wherein the covalent bond is a peptide bond.

35. The conjugate of claim 34 wherein the conjugate is a fusion protein having at its N-terminal end the variable region of an antibody capable of binding to the cell surface marker and at its C-terminal end the HIV env TCB domain.

36. A method for targeting cytotoxic lymphocytes to a selected cell surface marker which comprises preparing a conjugate of any one of claims 27-36.

37. An immunotoxin comprising:
a monoclonal antibody specifically binding a region of the TCB domain of an HIV env, and a toxin linked to said monoclonal antibody.

38. An immunotoxin according to claim 37 wherein said toxin is ricin A chain.

39. An immunotoxin according to claim 38 wherein said ricin A chain is deglycosylated.

40. An immunotoxin according to claim 38 or 39 wherein said ricin A chain is produced by recombinant DNA.

41. An immunotoxin according to any one of claims 37-40 wherein said monoclonal antibody is a murine monoclonal antibody.

42. An immunotoxin according to any one of claims 37-40 wherein said monoclonal antibody is a human monoclonal antibody.

43. An immunotoxin according to any one of claims 37-40 wherein said monoclonal antibody is a murine-human hybrid antibody.

44. An immunotoxin according to any one of claims 37-43 for killing HIV infected cells.

45. Use of an immunotoxin according to any one of claims 37-44 in the manufacture of a medicament for killing HIV infected cells.

46. Use of an immunotoxin comprising a monoclonal antibody specifically binding TCB domain, linked to a toxin, in the manufacture of a medicament for killing non-tumorigenic virus infected cells.

47. Use according to claim 46 wherein said toxin is ricin A chain.

## Claims (Claims for the following Contracting State(s): GR, ES)

1. Use of HIV env comprising the TCB domain in the manufacture of a medicament for the treatment of immunoinflammatory episodes.

2. Use according to claim 1 wherein the episode is associated with inflammatory bowel disease, rheumatoid arthritis, systemic lupus erythymatosis, juvenile-onset diabetes, Graves disease, myasthenia gravis, graft-versus-host rejection, and host-versus-graft rejection.

3. Use according to claim 1 or claim 2 wherein HIV env is rgp120.

4. Use according to any one of the preceding claims wherein the medicament is for administration in a therapeutic dose of about from 0.5 x 10⁻⁸ to 5 x 10⁻⁹ molar.

5. Use according to any one of the preceding claims wherein the medicament is a sustained release composition.

6. Use according to claim 5 wherein the sustained release composition is a polylactide.

7. Use according to any one of claims 1-4 wherein the medicament is for intravenous injection.

8. A method comprising production of a composition comprising the TCB domain of HIV env which is free of HIV immune epitopes ordinarily flanking the C-terminal or N-terminal ends of the TCB domain.

9. A method comprising production of a composition coprising the TCB domain of HIV env which is free of HIV env sequence flanking the C-terminus or N-terminus of the TCB domain.

10. The method of claim 9 wherein the TCB domain has the amino acid sequence

TEGSNNTEGSDTITLPCRIKQFI
NMWQEVGKAMYAPPISGQIRCSSNITGLLLTRDGGNNNNESEI.

11. The method of claim 9 wherein the TCB domain has the sequence CRIKQFINMWQEVGKAMYAPPISGQIRC.

12. The method of any one claims 8-11 wherein the composition is sterile and further contains a pharmacologically acceptable vehicle.

13. Use of the composition as producible by the method of any one of claims 8-12 in the manufacture of a medicament for the treatment of inflammatory episodes.

14. Use of a composition as producible by the method of any one of claims 8-13 in the manufacture of a medicament for the treatment of AIDS.

15. Use according to claim 14 wherein the medicament is a vaccine and includes an adjuvant.

16. Use according to claim 14 or claim 15 wherein the composition includes a tumor necrosis factor or an interferon.

17. Use according to claim 16 wherein the tumor necrosis factor is tumor necrosis factor-α and the interferon is interferon-γ.

18. A method comprising production of an antibody which is capable of blocking or binding the TCB domain

19. The method of claim 18 wherein the antibody is one which binds the TCB domain as defined in claim 10 or 11.

20. The method of claim 18 or 19 wherein the antibody is conjugated to a cytotoxin.

21. The method of claim 20 wherein the cytotoxin is ricin.

22. The method of claim 18 or 19 wherein the antibody is covalently bound to a detectable marker or a water insoluble matrix.

23. The method of any one of claims 18 to 22 further comprising the addition of a sterile pharmaceutically acceptable vehicle.

24. Use of an antibody as prepared by the method of any of claims 18 to 23 in the manufacture of a medicament for the treatment of HIV infection.

25. A method which comprises preparing a conjugate of (a) a substance capable of binding to a cell surface marker against which a cytotoxic T cell cytolytic response is desired and (b) the TCB domain of HIV env which is free of HIV immune epitopes ordinarily flanking the C-terminal or N-terminal ends of the TCB domain and which is capable of binding to the T4 receptor of helper lymphocytes.

26. The method of claim 25 wherein the cell surface marker is a protein.

27. The method of claim 25 or claim 26 wherein the conjugate comprises an HIV env fragment having the sequence

TEGSNNTEGSDTITLPCRIKQFINMWQEVGKAMYAPPISGQIRCSSNITGLLLTRD
GGNNNNESEI.

28. The method of any one of claims 25-27 wherein the substance capable of binding the cell surface marker comprises the variable region of an antibody.

29. The method of claim 28 wherein the antibody is capable of binding TGF-α.

30. The method of claim 25 wherein the marker binding substance is a hapten.

31. The method of any one of claims 25-30 wherein the substance capable of binding to the cell surface marker and the TCB domain are cross-linked by a covalent bond.

32. The method of claim 31 wherein the covalent bond is a peptide bond.

33. The method of claim 32 wherein the conjugate is a fusion protein having at its N-terminal end the variable region of an antibody capable of binding to the cell surface marker and at its C-terminal end the HIV env TCB domain.

34. A method comprising production of the conjugate as defined in any one of claims 25-33.

35. A method comprising production of an immunotoxin comprising:
a monoclonal antibody specifically binding a region of the TCB domain of an HIV env, and a toxin linked to said monoclonal antibody.

36. A method according to claim 35 wherein said toxin is ricin A chain.

37. A method according to claim 36 wherein said ricin A chain is deglycosylated.

38. A method according to claim 36 or 37 wherein said ricin A chain is produced by recombinant DNA.

39. A method according to any one of claims 35-38 wherein said monoclonal antibody is a murine monoclonal antibody.

40. A method according to any one of claims 35-38 wherein said monoclonal antibody is a human monoclonal antibody.

41. A method according to any one of claims 35-38 wherein said monoclonal antibody is a murine-human hybrid antibody.

42. Use of an immunotoxin as producible by the method of any one of claims 35-41 in the manufacture of a medicament for killing HIV infected cells.

43. Use of an immunotoxin comprising a monoclonal antibody specifically binding TCB domain, linked to a toxin, in the manufacture of a medicament for killing non-tumorigenic virus infected cells.

44. Use according to claim 43 wherein said toxin is ricin A chain.

## Patentansprüche (Patentansprüche für folgende(n) Vertragsstaat(en): DE, GB, FR, IT, NL, SE, CH, AT, BE, LU, LI)

1. Verwendung von die TCB-Domäne umfassendem HIV-env bei der Herstellung eines Medikaments zur Behandlung von Immunentzündungsepisoden.

2. Verwendung nach Anspruch 1, worin die Episode mit entzündlicher Darmerkankung, rheumatoider Arthritis, systemischem Lupus erythematodes , Jugenddiabetes, Basedow-Krankheit, Myasthenia gravis, Transplantat gegenüber Wirt-Abstoßung und Wirt gegenüber Transplantat-Abstoßung einhergeht.

3. Verwendung nach Anspruch 1 oder 2, worin HIV-env rgp120 ist.

4. Verwendung nach einem der vorhergehenden Ansprüche, worin das Medikament zur Verabreichung in einer therapeutischen Dosis von 0,5 x 10⁻⁸ bis 5 x 10⁻⁹ mol/l vorgesehen ist.

5. Verwendung nach einem der vorhergehenden Ansprüche, worin das Medikament eine Zusammensetzung mit Langzeitfreisetzung ist.

6. Verwendung nach Anspruch 5, worin die Zusammensetzung mit Langzeitfreisetzung ein Polylaktid ist.

7. Verwendung nach einem der Ansprüche 1-4, worin das Medikament zur intravenösen Injektion dient.

8. Zusammensetzung, umfassend die TCB-Domäne von HIV-env, die frei von HIV-Immunepitopen ist, die normalerweise das C- oder N-terminale Ende der TCB-Domäne flankieren.

9. Zusammensetzung, umfassend die TCB-Domäne von HIV-env, die frei von HIV-env-Sequenz ist, die den C-oder N-Terminus der TCB-Domäne flankiert.

10. Zusammensetzung nach Anspruch 9, worin die TCB-Domäne die folgende Aminosäuresequenz aufweist:

TEGSNNTEGSDTITLPCRIKQFINMWQEVGKAMYAPPISGQIRCSSNITGLLLTRDGGNNN
NESEI.

11. Zusammensetzung nach Anspruch 9, worin die TCB-Domäne die Sequenz CRIKQFINMWQEVGKAMYAPPISGQIRC besitzt.

12. Zusammensetzung nach einem der Ansprüche 8-11, die steril ist und weiters ein pharmakologisch annehmbares Vehikel enthält.

13. Zusammensetzung nach einem der Ansprüche 8-12 zur pharmazeutischen Verwendung.

14. Verwendung der Zusammensetzung nach einem der Ansprüche 8-13 bei der Herstellung eines Medikaments zur Behandlung von Entzündungsepisoden.

15. Verwendung einer Zusammensetzung nach einem der Ansprüche 8-13 bei der Herstellung eines Medikaments zur Behandlung von AIDS.

16. Verwendung nach Anspruch 15, worin das Medikament ein Impfstoff ist und ein Adjuvans enthält.

17. Verwendung nach Anspruch 15 oder 16, worin die Zusammensetzung einen Tumornekrosefaktor oder ein Interferon enthält.

18. Verwendung nach Anspruch 17, worin der Tumornekrosefaktor Tumornekrosefaktor-α und das Interferon Interferon-γ ist.

19. Monoklonaler Antikörper, der die TCB-Domäne blockieren oder binden kann.

20. Antikörper nach Anspruch 19, der sich an die in Anspruch 10 oder 11 definierten TCB-Domänen bindet.

21. Antikörper nach Anspruch 19 oder 20, der mit einem Zytotoxin konjugiert ist.

22. Antikörper nach Anspruch 21, worin das Zytotoxin Ricin ist.

23. Antikörper nach Anspruch 19 oder 20, der an einen nachweisbaren Marker oder eine wasserunlösliche Matrix kovalent gebunden ist.

24. Antikörper nach einem der Ansprüche 19-23 in einem sterilen, pharmazeutisch akzeptablen Vehikel.

25. Antikörper nach einem der Ansprüche 19-24 zur Behandlung von HIV-Infektion.

26. Verwendung des Antikörpers nach Anspruch 19 oder 20 bei der Herstellung eines Medikaments zur Behandlung von HIV-Infektion.

27. Konjugat von (a) einer Substanz, die einen Zelloberflächenmarker einer Zellpopulation, gegen die eine zytolytische Reaktion zytotoxischer T-Zellen erwünscht ist, binden kann und (b) der TCB-Domäne von HIV-env, die frei von HIV-Immunepitopen ist, die normalerweise das C- oder N-terminale Ende der TCB-Domäne flankieren, die sich an den T4-Rezeptor von Helferlymphozyten binden kann.

28. Konjugat nach Anspruch 27, worin der Zelloberflächenmarker ein Protein ist.

29. Konjugat nach Anspruch 27 oder 28, worin das Konjugat ein HIV-env-Fragment mit nachstehender Sequenz umfaßt:

TEGSNNTEGSDTITLPCRIKQFINMWQEVGKAMYAPPISGQIRCSSNITGLLLTRDGGNNN
NESEI.

30. Konjugat nach einem der Ansprüche 27-29, worin die Substanz, die den Zellobrflächenmarker binden kann, die variable Region eines Antikörpers umfaßt.

31. Konjugat nach Anspruch 30, worin der Antikörper TGF-α binden kann.

32. Konjugat nach Anspruch 27, worin die Markerbindesubstanz ein Hapten ist.

33. Konjugat nach einem der Ansprüche 27-32, worin die Substanz, die den Zelloberflächenmarker binden kann, und die TCB-Domäne durch eine kovalente Bindung verknüpft sind.

34. Konjugat nach Anspruch 33, worin die kovalente Bindung eine Peptidbindung ist.

35. Konjugat nach Anspruch 34, worin das Konjugat ein Fusionsprotein ist, das an seinem N-terminalen Ende die variable Region eines Antikörpers, der sich an den Zelloberflächenmarker binden kann, und an seinem C-terminalen Ende die HIV-env-TCB-Domäne aufweist.

36. Verfahren zum Ausrichten zytotoxischer Lymphozyten auf einen ausgewählten Zelloberflächenmarker, umfassend das Herstellen eines Konjugats nach einem der Ansprüche 27-36.

37. Immuntoxin, umfassend einen monoklonalen Antikörper, der eine Region der TCB-Domäne eines HIV-env spezifisch bindet, und ein mit dem monoklonalen Antikörper verbundenes Toxin.

38. Immuntoxin nach Anspruch 37, worin das Toxin die Ricin A-Kette ist.

39. Immuntoxin nach Anspruch 38, worin die Ricin A-Kette deglykosyliert ist.

40. Immuntoxin nach Anspruch 38 oder 39, worin die Ricin A-Kette durch rekombinante DNA hergestellt wird.

41. Immuntoxin nach einem der Ansprüche 37-40, worin der monoklonale Antikörper ein monoklonaler Mäuseantikörper ist.

42. Immuntoxin nach einem der Ansprüche 37-40, worin der monoklonale Antikörper ein menschlicher monoklonaler Antikörper ist.

43. Immuntoxin nach einem der Ansprüche 37-40, worin der monoklonale Antikörper ein Mäuse-Mensch-Hybridantikörper ist.

44. Immuntoxin nach einem der Ansprüche 37-43 zum Töten HIV-infizierter Zellen.

45. Verwendung eines Immuntoxins nach einem der Ansprüche 37-44 bei der Herstellung eines Medikaments zum Abtöten HIV-infizierter Zellen.

46. Verwendung eines Immuntoxins umfassend einen monoklonalen Antikörper, der die TCB-Domäne spezifisch binden kann und der mit einem Toxin verbunden ist, bei der Herstellung eines Medikaments zum Abtöten nicht-tumorerzeugender, virusinfizierter Zellen.

47. Verwendung nach Anspruch 46, worin das Toxin die Ricin A-Kette ist.

## Patentansprüche (Patentansprüche für folgende(n) Vertragsstaat(en): GR, ES)

1. Verwendung von die TCB-Domäne umfassendem HIV-env bei der Herstellung eines Medikaments zur Behandlung von Immunentzündungsepisoden.

2. Verwendung nach Anspruch 1, worin die Episode mit entzündlicher Darmerkankung, rheumatoider Arthritis, systemischem Lupus erythematodes , Jugenddiabetes, Basedow-Krankheit, Myasthenia gravis, Transplantat gegenüber Wirt-Abstoßung und Wirt gegenüber Transplantat-Abstoßung einhergeht.

3. Verwendung nach Anspruch 1 oder 2, worin HIV-env rgp120 ist.

4. Verwendung nach einem der vorhergehenden Ansprüche, worin das Medikament zur Verabreichung in einer therapeutischen Dosis von 0,5 x 10⁻⁸ bis 5 x 10⁻⁹ mol/l vorgesehen ist.

5. Verwendung nach einem der vorhergehenden Ansprüche, worin das Medikament eine Zusammensetzung mit Langzeitfreisetzung ist.

6. Verwendung nach Anspruch 5, worin die Zusammensetzung mit Langzeitfreisetzung ein Polylaktid ist.

7. Verwendung nach einem der Ansprüche 1-4, worin das Medikament zur intravenösen Injektion dient.

8. Verfahren, umfassend die Herstellung einer Zusammensetzung umfassend die TCB-Domäne von HIV-env, die frei von HIV-Immunepitopen ist, die normalerweise das C-oder N-terminale Ende der TCB-Domäne flankieren.

9. Verfahren, umfassend die Herstellung einer Zusammensetzung umfassend die TCB-Domäne von HIV-env, die frei von HIV-env-Sequenz ist, die den C- oder N-Terminus der TCB-Domäne flankiert.

10. Verfahren nach Anspruch 9, worin die TCB-Domäne die folgende Aminosäuresequenz aufweist:

TEGSNNTEGSDTITLPCRIKQFINMWQEVGKAMYAPPISGQIRCSSNITGLLLTRDGGNNN
NESEI.

11. Verfahren nach Anspruch 9, worin die TCB-Domäne die Sequenz CRIKQFINMWQEVGKAMYAPPISGQIRC besitzt.

12. Verfahren nach einem der Ansprüche 8-11, worin die Zusammensetzung steril ist und weiters ein pharmakologisch annehmbares Vehikel enthält.

13. Verwendung der durch das Verfahren nach einem der Ansprüche 8-12 herstellbaren Zusammensetzung bei der Herstellung eines Medikaments zur Behandlung von Entzündungsepisoden.

14. Verwendung einer durch das Verfahren nach einem der Ansprüche 8-13 herstellbaren Zusammensetzung bei der Herstellung eines Medikaments zur Behandlung von AIDS.

15. Verwendung nach Anspruch 14, worin das Medikament ein Impfstoff ist und ein Adjuvans enthält.

16. Verwendung nach Anspruch 14 oder 15, worin die Zusammensetzung einen Tumornekrosefaktor oder ein Interferon enthält.

17. Verwendung nach Anspruch 16, worin der Tumornekrosefaktor Tumornekrosefaktor-α und das Interferon Interferon-γ ist.

18. Verfahren, umfassend die Herstellung eines Antikörpers, der die TCB-Domäne blockieren oder binden kann.

19. Verfahren nach Anspruch 19, worin der Antikörper einer ist, der sich an die in Anspruch 10 oder 11 definierte TCB-Domäne bindet.

20. Verfahren nach Anspruch 18 oder 19, worin der Antikörper mit einem Zytotoxin konjugiert ist.

21. Verfahren nach Anspruch 20, worin das Zytotoxin Ricin ist.

22. Verfahren nach Anspruch 18 oder 19, worin der Antikörper an einen nachweisbaren Marker oder eine wasserunlösliche Matrix kovalent gebunden ist.

23. Verfahren nach einem der Ansprüche 18-22, weiters umfassend die Zugabe eines sterilen, pharmazeutisch akzeptablen Vehikels.

24. Verwendung eines Antikörper, der nach dem Verfahren nach einem der Ansprüche 18 bis 23 hergestellt wird, bei der Herstellung eines Medikaments zur Behandlung von HIV-Infektion.

25. Verfahren, umfassend das Herstellen eines Konjugats von (a) einer Substanz, die einen Zelloberflächenmarker einer Zellpopulation, gegen die eine zytolytische Reaktion zytotoxischer T-Zellen erwünscht ist, binden kann und (b) der TCB-Domäne von HIV-env, die frei von HIV-Immunepitopen ist, die normalerweise das C- oder N-terminale Ende der TCB-Domäne flankieren, die sich an den T4-Rezeptor von Helferlymphozyten binden kann.

26. Verfahren nach Anspruch 25, worin der Zelloberflächenmarker ein Protein ist.

27. Verfahren nach Anspruch 25 oder 26, worin das Konjugat ein HIV-env-Fragment mit nachstehender Sequenz umfaßt:

TEGSNNTEGSDTITLPCRIKQFINMWQEVGKAMYAPPISGQIRCSSNITGLLLTRDGGNNN
NESEI.

28. Verfahren nach einem der Ansprüche 25-27, worin die Substanz, die den Zelloberflächenmarker binden kann, die variable Region eines Antikörpers umfaßt.

29. Verfahren nach Anspruch 28, worin der Antikörper TGF-α binden kann.

30. Verfahren nach Anspruch 25, worin die Markerbindesubstanz ein Hapten ist.

31. Verfahren nach einem der Ansprüche 25-30, worin die Substanz, die den Zelloberflächenmarker binden kann, und die TCB-Domäne durch eine kovalente Bindung verknüpft sind.

32. Verfahren nach Anspruch 31, worin die kovalente Bindung eine Peptidbindung ist.

33. Verfahren nach Anspruch 32, worin das Konjugat ein Fusionsprotein ist, das an seinem N-terminalen Ende die variable Region eines Antikörpers, der sich an den Zelloberflächenmarker binden kann, und an seinem C-terminalen Ende die HIV-env-TCB-Domäne aufweist.

34. Verfahren, umfassend die Herstellung des Konjugats nach einem der Ansprüche 25-33.

35. Verfahren, umfassend die Herstellung eines Immuntoxins, das einen monoklonalen Antikörper, der eine Region der TCB-Domäne eines HIV-env spezifisch bindet, und ein mit dem monoklonalen Antikörper verbundenes Toxin umfaßt.

36. Verfahren nach Anspruch 35, worin das Toxin die Ricin A-Kette ist.

37. Verfahren nach Anspruch 36, worin die Ricin A-Kette deglykosyliert ist.

38. Verfahren nach Anspruch 36 oder 37, worin die Ricin A-Kette durch rekombinante DNA hergestellt wird.

39. Verfahren nach einem der Ansprüche 35-38, worin der monoklonale Antikörper ein monoklonaler Mäuseantikörper ist.

40. Verfahren nach einem der Ansprüche 35-38, worin der monoklonale Antikörper ein menschlicher monoklonaler Antikörper ist.

41. Verfahren nach einem der Ansprüche 35-38, worin der monoklonaler Antikörper ein Mäuse-Human-Hybridantikörper ist.

42. Verwendung eines durch das Verfahren nach einem der Ansprüche 35-41 herstellbaren Immuntoxins bei der Herstellung eines Medikaments zum Abtöten HIV-infizierter Zellen.

43. Verwendung eines Immuntoxins umfassend einen monoklonalen Antikörper, der TCB-Domäne spezifisch bindet, und der mit einem Toxin verbunden ist, bei der Herstellung eines Medikaments zum Abtöten nicht-tumorerzeugender, virusinfizierter Zellen.

44. Verwendung nach Anspruch 43, worin das Toxin die Ricin A-Kette ist.

## Revendications (Revendications pour l'(les) Etat(s) contractant(s) suivant(s): DE, GB, FR, IT, NL, SE, CH, AT, BE, LU, LI)

1. Utilisation de env de HIV comprenant le domaine TCB dans la production d'un médicament destiné au traitement d'épisodes immuno-inflammatoires.

2. Utilisation suivant la revendication 1, dans laquelle l'épisode est associée à une maladie intestinale inflammatoire, l'arthrite rhumatoïde, le lupus érythémateux généralisé, le diabète juvénile, le goître exophtalmique, la myasthénie grave, un rejet du greffon par l'hôte et un rejet de l'hôte par le greffon.

3. Utilisation suivant la revendication 1 ou la revendication 2, dans laquelle le env de HIV consiste en rgp120.

4. Utilisation suivant l'une quelconque des revendications précédentes, dans laquelle le médicament est destiné à l'administration à une dose thérapeutique d'environ 0,5x10⁻⁸ à 5x10⁻⁹ molaire.

5. Utilisation suivant l'une quelconque des revendications précédentes, dans laquelle le médicament est une composition à libération prolongée.

6. Utilisation suivant la revendication 5, dans laquelle la composition à libération prolongée est un polylactide.

7. Utilisation suivant l'une quelconque des revendications 1 à 4, dans laquelle le médicament est destiné à l'injection intraveineuse.

8. Composition comprenant le domaine TCB de env de HIV qui est dépourvu des épitopes immuns du HIV habituellement adjacents aux extrémités C-terminales ou N-terminales du domaine TCB.

9. Composition comprenant le domaine TCB de env de HIV qui est dépourvu de séquence de env de HIV adjacente à l'extrémité C-terminale ou l'extrémité N-terminale du domaine TCB.

10. Composition suivant la revendication 9, dans laquelle le domaine TCB possède la séquence d'amino-acides

TEGSNNTEGSDTITLPCRIKQFI
NMWQEVGKAMYAPPISGQIRCSSNITGLLLTRDGGNNNNESEI.

11. Composition suivant la revendication 9, dans laquelle le domaine TCB possède la séquence
CRIKQFINMWQEVGKAMYAPPISGQIRC.

12. Composition suivant l'une quelconque des revendications 8 à 11, qui est stérile et qui contient en outre un véhicule pharmacologiquement acceptable.

13. Composition suivant l'une quelconque des revendications 8 à 12, à usage pharmaceutique.

14. Utilisation de la composition suivant l'une quelconque des revendications 8 à 13, dans la production d'un médicament destiné au traitement d'épisodes inflammatoires.

15. Utilisation d'une composition suivant l'une quelconque des revendications 8 à 13, dans la production d'un médicament destiné au traitement du SIDA.

16. Utilisation suivant la revendication 15, dans laquelle le médicament est un vaccin et renferme un adjuvant.

17. Utilisation suivant la revendication 15 ou la revendication 16, dans laquelle la composition comprend un facteur de nécrose tumorale ou un interféron.

18. Utilisation suivant la revendication 17, dans laquelle le facteur de nécrose tumorale est le facteur de nécrose tumorale α, et l'interféron consiste en interféron-γ.

19. Anticorps monoclonal qui est capable de bloquer ou de fixer le domaine TCB.

20. Anticorps suivant la revendication 19, qui se lie au domaine TCB répondant à la définition suivant la revendication 10 ou 11.

21. Anticorps suivant la revendication 19 ou 20, qui est conjugué à une cytotoxine.

22. Anticorps suivant la revendication 21, dans lequel la cytotoxine est la ricine.

23. Anticorps suivant la revendication 19 ou 20, qui est lié par covalence à un marqueur détectable ou une matrice insoluble dans l'eau.

24. Anticorps suivant l'une quelconque des revendications 19 à 23, dans un véhicule pharmaceutiquement acceptable stérile.

25. Anticorps suivant l'une quelconque des revendications 19 à 24, pour le traitement d'une infection par le HIV.

26. Utilisation de l'anticorps suivant la revendication 19 ou 20, dans la production d'un médicament destiné au traitement d'une infection par le HIV.

27. Conjugué (a) d'une substance capable de se lier à un marqueur de surface cellulaire d'une population de cellules contre lesquelles une réponse cytolytique de lymphocytes T cytotoxiques est désirée, et (b) du domaine TCB de env de HIV qui est dépourvu des épitopes immuns de HIV habituellement adjacents aux extrémités C-terminales ou N-terminales du domaine TCB qui est capable de se lier au récepteur T4 de lymphocytes auxiliaires.

28. Conjugué suivant la revendication 27, dans lequel le marqueur de surfaces cellulaires est une protéine.

29. Conjugué suivant la revendication 27 ou la revendication 28, qui comprend un fragment env de HIV ayant la séquence

TEGSNNTEGSDTITLPCRIKQFINMWQEVGKAMYAPPISGQIRCSSNITGLLLTRD

GGNNNNESEI.

30. Conjugué suivant l'une quelconque des revendications 27 à 29, dans lequel la substance capable de se lier au marqueur de surface cellulaire comprend la région variable d'un anticorps.

31. Conjugué suivant la revendication 30, dans lequel l'anticorps est capable de se lier au TGF-α.

32. Conjugué suivant la revendication 27, dans lequel la substance se liant au marqueur est un haptène.

33. Conjugué suivant l'une quelconque des revendications 27 à 32, dans lequel la substance capable de se lier au marqueur de surface cellulaire et le domaine TCB sont réticulés par une liaison covalente.

34. Conjugué suivant la revendication 33, dans lequel la liaison covalente est une liaison peptidique.

35. Conjugué suivant la revendication 34, qui est une protéine de fusion ayant à son extrémité N-terminale la région variable d'un anticorps capable de se lier au marqueur de surface cellulaire et à son extrémité C-terminale le domaine TCB de env de HIV.

36. Procédé pour diriger des lymphocytes cytotoxiques vers une cible consistant en un marqueur de surface cellulaire choisi, qui comprend la préparation d'un conjugué suivant l'une quelconque des revendications 27 à 36.

37. Immunotoxine comprenant :
un anticorps monoclonal se liant spécifiquement à une région du domaine TCB d'un env de HIV, et une toxine liée audit anticorps monoclonal.

38. Immunotoxine suivant la revendication 37, dans laquelle la toxine est la chaîne A de la ricine.

39. Immunotoxine suivant la revendication 38, dans laquelle la chaîne A de la ricine est déglycosylée.

40. Immunotoxine suivant la revendication 38 ou 39, dans laquelle la chaîne A de la ricine est produite au moyen d'un ADN recombinant.

41. Immunotoxine suivant l'une quelconque des revendications 37 à 40, dans laquelle l'anticorps monoclonal est un anticorps monoclonal murin.

42. Immunotoxine suivant l'une quelconque des revendications 37 à 40, dans laquelle l'anticorps monoclonal est un anticorps monoclonal humain.

43. Immunotoxine suivant l'une quelconque des revendications 37 à 40, dans laquelle l'anticorps monoclonal est un anticorps hybride murin-humain.

44. Immunotoxine suivant l'une quelconque des revendications 37 à 43 pour la destruction de cellules infectées par le HIV.

45. Utilisation d'une immunotoxine suivant l'une quelconque des revendications 37 à 44 dans la production d'un médicament destiné à détruire des cellules infectées par le HIV.

46. Utilisation d'une immunotoxine comprenant un anticorps monoclonal se liant spécifiquement au domaine TCB, lié à une toxine, dans la production d'un médicament destiné à détruire des cellules infectées par des virus non tumorigènes.

47. Utilisation suivant la revendication 46, dans laquelle la toxine est la chaîne A de la ricine.

## Revendications (Revendications pour l'(les) Etat(s) contractant(s) suivant(s): GR, ES)

1. Utilisation de env de HIV comprenant le domaine TCB dans la production d'un médicament destiné au traitement d'épisodes immuno-inflammatoires.

2. Utilisation suivant la revendication 1, dans laquelle l'épisode est associée à une maladie intestinale inflammatoire, l'arthrite rhumatoïde, le lupus érythémateux disséminé, le diabète juvénile, le goître exophtalmique, la myasthénie grave, le rejet du greffon par l'hôte et le rejet de l'hôte par le greffon.

3. Utilisation suivant la revendication 1 ou la revendication 2, dans laquelle le env de HIV consiste en rgp120.

4. Utilisation suivant l'une quelconque des revendications précédentes, dans laquelle le médicament est destiné à être administrée à une dose thérapeutique d'environ 0,5x10⁻⁸ à 5x10⁻⁹ molaire.

5. Utilisation suivant l'une quelconque des revendications précédentes, dans laquelle le médicament est une composition à libération prolongée.

6. Utilisation suivant la revendication 5, dans laquelle la composition à libération prolongée est un polylactide.

7. Utilisation suivant l'une quelconque des revendications 1 à 4, dans laquelle le médicament est destiné à l'injection intraveineuse.

8. Procédé comprenant la production d'une composition comprenant le domaine TCB de env de HIV qui est dépourvu d'épitopes immuns du HIV habituellement adjacents aux extrémités C-terminales ou N-terminales du domaine TCB.

9. Procédé comprenant la production d'une composition comprenant le domaine TCB de env de HIV qui est dépourvu de la séquence de env de HIV flanquant l'extrémité C-terminale ou l'extrémité N-terminale du domaine TCB.

10. Procédé suivant la revendication 9, dans lequel le domaine TCB a la séquence d'amino-acides

TEGSNNTEGSDTITLPCRIKQFINMWQEVGKAMYAPPISGQIRCSSNITGLLLTRD
GGNNNNESEI.

11. Procédé suivant la revendication 9, dans lequel le domaine TCB possède la séquence
CRIKQFINMWQEVGKAMYAPPISGQIRC.

12. Procédé suivant l'une quelconque des revendications 8 à 11, dans lequel la composition est stérile et contient en outre un véhicule pharmacologiquement acceptable.

13. Utilisation de la composition pouvant être produite par le procédé suivant l'une quelconque des revendications 8 à 12, dans la production d'un médicament destiné au traitement d'épisodes inflammatoires.

14. Utilisation d'une composition pouvant être produite par le procédé suivant l'une quelconque des revendications 8 à 13, dans la production d'un médicament destiné au traitement du SIDA.

15. Utilisation suivant la revendication 14, dans laquelle le médicament est un vaccin et comprend un adjuvant.

16. Utilisation suivant la revendication 14 ou la revendication 15, dans laquelle la composition comprend un facteur de nécrose tumorale ou un interféron.

17. Utilisation suivant la revendication 16, dans laquelle le facteur de nécrose tumorale est le facteur de nécrose tumorale α et l'interféron consiste en interféron-γ.

18. Procédé comprenant la production d'un anticorps qui est capable de bloquer ou de fixer le domaine TCB.

19. Procédé suivant la revendication 18, dans lequel l'anticorps est un anticorps qui se lie au domaine TCB répondant à la définition suivant la revendication 10 ou 11.

20. Procédé suivant la revendication 18 ou 19, dans lequel l'anticorps est conjugué à une cytotoxine.

21. Procédé suivant la revendication 20, dans lequel la cytotoxine est la ricine.

22. Procédé suivant la revendication 18 ou 19, dans lequel l'anticorps est lié par covalence à un marqueur détectable ou une matrice insoluble dans l'eau.

23. Procédé suivant l'une quelconque des revendications 18 à 22, comprenant en outre l'addition d'un véhicule pharmaceutiquement acceptable stérile.

24. Utilisation d'un anticorps préparé par le procédé suivant l'une quelconque des revendications 18 à 23 dans la production d'un médicament destiné au traitement d'une infection par le HIV.

25. Procédé qui comprend la préparation d'un conjugué (a) d'une substance capable de se lier à un marqueur de surface cellulaire contre lequel une réponse cytolytique de lymphocytes T cytotoxiques est désirée, et (b) du domaine TCB de env de HIV qui est dépourvu d'épitopes immuns du HIV habituellement adjacents aux extrémités C-terminales ou N-terminales du domaine TCB et qui est capable de se lier au récepteur T4 de lymphocytes auxiliaires.

26. Procédé suivant la revendication 25, dans lequel le marqueur de surface cellulaire est une protéine.

27. Procédé suivant la revendication 25 ou la revendication 26, dans lequel le conjugué comprend un fragment env de HIV ayant la séquence

TEGSNNTEGSDTITLPCRIKQFINMWQEVGKAMYAPPISGQIRCSSNITGLLLTRD
GGNNNNESEI.

28. Procédé suivant l'une quelconque des revendications 25 à 27, dans lequel la substance capable de se lier au marqueur de surface cellulaire comprend la région variable d'un anticorps.

29. Procédé suivant la revendication 28, dans lequel l'anticorps est capable de se lier au TGF-α.

30. Procédé suivant la revendication 25, dans lequel la substance se liant au marqueur est un haptène.

31. Procédé suivant l'une quelconque des revendications 25 à 30, dans lequel la substance capable de se lier au marqueur de surface cellulaire et le domaine TCB sont réticulés par une liaison covalente.

32. Procédé suivant la revendication 31, dans lequel la liaison covalente est une liaison peptidique.

33. Procédé suivant la revendication 32, dans lequel le conjugué est une protéine de fusion ayant à son extrémité N-terminale la région variable d'un anticorps capable de se lier au marqueur de surface cellulaire et à son extrémité C-terminale le domaine TCB de env de HIV.

34. Procédé comprenant la production d'un conjugué répondant à la définition suivant l'une quelconque des revendications 25 à 33.

35. Procédé comprenant la production d'une immunotoxine comprenant :
un anticorps monoclonal se liant spécifiquement à une région du domaine TCB d'un env de HIV, et une toxine liée audit anticorps monoclonal.

36. Procédé suivant la revendication 35, dans lequel la toxine est la chaîne A de la ricine.

37. Procédé suivant la revendication 36, dans lequel la chaîne A de la ricine est déglycosylée.

38. Procédé suivant la revendication 36 ou 37, dans lequel la chaîne A de la ricine est produite au moyen d'un ADN recombinant.

39. Procédé suivant l'une quelconque des revendications 35 à 38, dans lequel l'anticorps monoclonal est un anticorps monoclonal murin.

40. Procédé suivant l'une quelconque des revendications 35 à 38, dans lequel l'anticorps monoclonal est un anticorps monoclonal humain.

41. Procédé suivant l'une quelconque des revendications 35 à 38, dans lequel l'anticorps monoclonal est un anticorps hybride murin-humain.

42. Utilisation d'une immunotoxine pouvant être produite par le procédé suivant l'une quelconque des revendications 35 à 41 dans la production d'un médicament destiné à la destruction de cellules infectées par le HIV.

43. Utilisation d'une immunotoxine comprenant un anticorps monoclonal se liant spécifiquement au domaine TCB, lié à une toxine, dans la production d'un médicament destiné à la destruction de cellules infectées par des virus non tumorigènes.

44. Utilisation suivant la revendication 43, dans laquelle la toxine est la chaîne A de la ricine.
